# EUROPÄISCHE PATENTSCHRIFT

(11) **EP 2 410 971 B1**
(45) Veröffentlichungstag und Bekanntmachung des Hinweises auf die Patenterteilung: **16.08.2017**
(21) Anmeldenummer: 10709840.2
(22) Anmeldetag: 19.03.2010
(51) Int. Cl.: A61K 6/09, C08G 18/67, C08G 18/75, C08G 18/76, C08G 18/79, C08G 83/00, C08G 18/10, C08L 75/04, C08G 18/08

(54) **STRAHLUNGSHÄRTBARE HOCHFUNKTIONELLE POLYURETHAN(METH)ACRYLATE**
RADIATION-CURING, HIGHLY FUNCTIONAL POLYURETHANE (METH)ACRYLATE
(METH)ACRYLATES DE POLYURÉTHANE À HAUTE FONCTIONNALITÉ DURCISSABLES PAR RAYONNEMENT

(30) Priorität: 24.03.2009 EP 09156061
(43) Veröffentlichungstag der Anmeldung: 01.02.2012
(73) Patentinhaber: BASF SE, 67056 Ludwigshafen am Rhein (DE)
(72) Erfinder: BRUCHMANN, Bernd, 67251 Freinsheim (DE); BECK, Erich, 68526 Ladenburg (DE); PAULUS, Wolfgang, 55270 Ober-Olm (DE)
(74) Vertreter: BASF IP Association
(86) Internationale Anmeldenummer: PCT/EP2010/053609
(87) Internationale Veröffentlichungsnummer: WO 2010/108863

(56) Entgegenhaltungen:
- EP-A1- 1 714 633
- EP-A2- 1 134 247
- WO-A1-03/091347
- WO-A1-2005/014679
- WO-A1-2008/075806
- DE-A1-102004 040 398
- DE-A1-102004 040 419
- US-A1- 2006 264 598
- ZHU S ET AL: "Flame retardant mechanism of hyperbranched polyurethane acrylates used for UV curable flame retardant coatings" POLYMER DEGRADATION AND STABILITY, BARKING, GB LNKD- DOI:10.1016/S0141-3910(01)00257-9, Bd. 75, Nr. 3, 1. Januar 2002 (2002-01-01) , Seiten 543-547, XP004334223 ISSN: 0141-3910
- SHENG-WU ZHU, WEN-FANG SHI: "Synthesis and photopolymerization of hyperbranched polyurethane acrylates applied to UV curable flame retardant coatings" POLYMER INTERNATIONAL, Bd. 51, Nr. 3, 31. Januar 2002 (2002-01-31), Seiten 223-227, XP002584864

## Beschreibung

Die vorliegende Erfindung betrifft Gemische, enthaltend gezielt aufgebaute, strahlungshärtbare, hochfunktionelle, hoch- oder hyperverzweigte Polyurethan(meth)acrylate, Verfahren zu deren Herstellung sowie deren Verwendung.

Dendrimere sowie hochfunktionelle, hoch- oder hyperverzweigte Polyisocyanate sind in der Literatur beschrieben. Aus diesen strahlungshärtbare Polyurethan(meth)acrylate herzustellen, ist beispielsweise bekannt aus EP 1134247 A2.

WO 02/062901 offenbart Gemische aus dendritischen Polyester(meth)acrylaten, einem reaktiven Lösungsmittel und anorganischem Füllmaterial aus Nanomaterialien zur Anwendung als Zahnfüllungsmaterial.

EP 1714633 A1 beschreibt Gemische aus dendritischen Polyurethanmethacrylaten mit anderen strahlungshärtbaren Reaktivverdünnern zur Anwendung in Dentalmassen.

Die beschriebenen Gemische werden jeweils durch Vermischung der fertigen Komponenten, nämlich polyfunktioneller (Meth)acrylate und Reaktivverdünner, hergestellt.

Nachteilig daran ist, daß die beschriebenen (Meth)acrylate aufgrund ihrer hohen Funktionalität eine ausgeprägte Tendenz zur Vernetzung aufweisen, die ihre Viskosität erhöht und die für die gewünschte Reaktion zur Verfügung stehende Funktionalität herabsetzt. Die beschriebenen Herstellungsverfahren vermögen diese ausgeprägte Vernetzungstendenz nicht verringern.

Aus DE 10 2004 040 398 A1 ist bekannt, hyperverzweigte Polyurethane für die Verwendung in wäßrigen Tinten mit reaktiven Acrylaten oder di- oder multifunktionellen Acrylaten zu vermischen oder in deren Gegenwart zu synthetisieren. In den explizit offenbarten Beispielen werden ausschließlich mit NCO-Gruppen reaktive Acrylate eingesetzt.

Nachteilig an dem Verfahren ist, daß di- oder multifunktionelle Acrylate nur eine geringe Löslichkeit bzw. Mischbarkeit im wäßrigen Medium aufweisen, so daß sich die erhaltenen wäßrigen Mischungen leicht entmischen.

Der vorliegende Erfindung lag daher die Aufgabe zugrunde, ein Verfahren zur Herstellung von strahlungshärtbaren hochfunktionellen Polyurethan(meth)acrylaten bereitzustellen, die aufgrund ihres definierten Aufbaus vorteilhafte Eigenschaften, wie hohe Funktionalität, hohe Reaktivität, geringe Viskosität und/oder gute Löslichkeit, in sich vereinen können. Es lag weiterhin die Aufgabe zugrunde, ein Verfahren zur Herstellung von strahlungshärtbaren hochfunktionellen Polyurethan(meth)acrylaten bereitzustellen, mit dem die Tendenz zur Vernetzung zurückgedrängt werden kann.

Die Aufgabe wird gelöst durch ein Verfahren zur Herstellung von strahlungshärtbaren, hochfunktionellen, hoch- oder hyperverzweigten Polyurethan(meth)acrylaten (U), umfassend die Reaktionsschritte
(i) Herstellen eines Additionsproduktes (A), das eine oder mehrere Isocyanatgruppen enthält und mindestens eine mit Isocyanat reaktive Gruppe enthält, durch Umsetzen eines
   (I) (a1) Di- und/oder
   (I) (a2) Polyisocyanats
   mit
   (b1) mindestens einer Verbindung mit mindestens drei mit Isocyanat reaktiven Gruppen
      und/oder
   (b2) mindestens einer Verbindung mit zwei mit Isocyanat reaktiven Gruppen,
   wobei mindestens eine der Komponenten (a) oder (b) funktionelle Gruppen mit gegenüber den funktionellen Gruppen der anderen Komponente unterschiedlicher Reaktivität aufweist,
   wobei die reaktiveren Gruppen aus den Verbindungen (a) und (b) in Schritt (i) im wesentlichen abreagieren, und
   wobei das Umsetzungsverhältnis so gewählt wird, dass im Mittel das Additionsprodukt (A) mindestens eine mit Isocyanat reaktive Gruppe und eine oder mehrere Isocyanatgruppen enthält,
(ii) gegebenenfalls intermolekulare Additionsreaktion des Additionsprodukts (A) aus (i) zu einem Polyadditionsprodukt (P), das eine oder mehrere Isocyanatgruppen enthält und mindestens eine mit Isocyanat reaktive Gruppe enthalten kann,
(iii) gegebenenfalls Umsetzen des Additionsproduktes (A) aus (i) oder (P) aus (ii) mit mindestens einem Monoisocyanat und/oder mindestens einem Di- oder Polyisocyanat (I)(a1) oder (I)(a2) und/oder mindestens einem Di- oder Polyisocyanat (II), das vom Di- oder Polyisocyanat (I) verschieden ist, und
(iv) Umsetzen des Additionsproduktes (A) aus (i) und/oder des Polyadditionsproduktes (P) aus (ii) und/oder des Reaktionsproduktes aus (iii) mit mindestens einer Verbindung (c), die mindestens eine, bevorzugt genau eine gegenüber Isocyanat reaktive Gruppen und mindestens eine (Meth)acrylatgruppe aufweist,
in dem bei der Herstellung während des Reaktionsschrittes (i) mindestens ein Polyether(meth)acrylat oder ein (Meth)acrylat eines Di-, Tri- oder Tetraols (V) anwesend ist, das mindestens eine strahlungshärtbare Gruppe aufweist und das gegenüber den Reaktionspartnern aus Reaktionschritt (i) inert ist, wobei das mindestens eine Polyether(meth)acrylat oder (Meth)acrylat eines Di-, Tri- oder Tetraols (V) ein mittleres Molekulargewicht bis zu 1000 g/mol aufweist.

Die Schritte (ii), (iii) und (iv) können dem Schritte (i) in beliebiger Reihenfolge folgen. Dabei sind die Schritte (ii) und (iii) optional, der Schritt (i) kann gegebenenfalls auch mehrmals erfolgen.

Bevorzugte Abfolgen der Reaktionsschritte sind (i) - (ii) - (iii) - (iv) und (i) - (iii) - (iv).

Zwischen den einzelnen Reaktionsschritten können jeweils Aufarbeitungs- oder Aufreinigungsschritte stattfinden, wie beispielsweise Extraktion, Wäsche, Strippung, Destillation oder Filtration. Falls erforderlich kann das Reaktionsgemisch einer Entfärbung, beispielsweise durch Behandlung mit Aktivkohle oder Metalloxiden, wie z.B. Aluminiumoxid, Siliciumoxid, Magnesiumoxid, Zirkonoxid, Boroxid oder Gemischen davon, in Mengen von beispielsweise 0,1 - 50 Gew%, bevorzugt 0,5 bis 25 Gew%, besonders bevorzugt 1 - 10 Gew% bezogen auf das Reaktionsgemisch bei Temperaturen von beispielsweise 10 bis 100 °C, bevorzugt 20 bis 80 °C und besonders bevorzugt 30 bis 60 °C unterworfen werden. Dies kann durch Zugabe des pulver- oder granulatförmigen Entfärbungsmittels zum Reaktionsgemisch und nachfolgender Filtration oder durch Überleiten des Reaktionsgemisches über eine Schüttung des Entfärbungsmittels in Form beliebiger, geeigneter Formkörper erfolgen.

In einer bevorzugten Ausführungsform der vorliegenden Erfindung werden die einzelnen Reaktionsschritte jedoch in demselben Reaktor durchgeführt, besonders bevorzugt ohne zwischenzeitliche Aufarbeitungs- oder Aufreinigungsschritte. Eine Aufarbeitung oder Aufreinigung erfolgt in diesem Fall gegebenenfalls erst nach dem letzten Reaktionsschritt (iv).

Ein weiterer Gegenstand der Erfindung sind die nach diesem Verfahren hergestellten strahlungshärtbaren Gemische, enthaltend hochfunktionelle, hoch- oder hyperverzweigte Polyurethan(meth)acrylate (U) mit Verbindungen (V).

Ein Gegenstand der Erfindung ist ferner die Verwendung der erfindungsgemäßen Gemische, enthaltend strahlungshärtbare hochfunktionelle, hoch- oder hyperverzweigte Polyurethan(meth)acrylate (U) und Verbindungen (V) als Bausteine zur Herstellung von Lacken, Überzügen, Beschichtungsmassen, Formmassen und Dentalmassen, bevorzugt als Bausteine zur Herstellung von Dentalmassen.

Hyperverzweigte Polyisocyanate und -(meth)acrylate können auf der einen Seite ausgehend von einem Zentralmolekül analog zu Dendrimeren, jedoch mit uneinheitlicher Kettenlänge der Äste aufgebaut sein. Sie können auf der anderen Seite auch linear, mit funktionellen Seitengruppen, aufgebaut sein oder aber, als Kombination der beiden Extreme, lineare und verzweigte Molekülteile aufweisen. Zur Definition von dendrimeren und hyperverzweigten Polymeren siehe auch P.J. Flory, J. Am. Chem. Soc. 1952, 74, 2718 und H. Frey et al., Chemistry - A European Journal, 2000, 6, No. 14, 2499.

Unter "hyperverzweigt" wird im Zusammenhang mit der vorliegenden Erfindung verstanden, dass der Verzweigungsgrad (Degree of Branching, DB), das ist der Quotient aus der Summe der mittleren Anzahl von dendritischen Verknüpfungen und der terminalen Einheiten zum einen und der Summe der mittleren Zahl der Gesamtverknüpfungen (dendritische, lineare und terminale Verknüpfungen) zum anderen, multipliziert mit 100, 10 bis 99,9 %, bevorzugt 10 bis 90 % und insbesondere 20 bis 80 % beträgt.

Unter "dendrimer" wird im Zusammenhang mit der vorliegenden Erfindung verstanden, daß der Verzweigungsgrad 99,9 - 100% beträgt. Zur Definition des "Degree of Branching" siehe H. Frey et al., Acta Polym. 1997, 48, 30 - 35.

Unter einem hochfunktionellen Polyurethan(meth)acrylat (U) ist im Rahmen dieser Erfindung ein Polyurethan(meth)acrylat zu verstehen, das mindestens drei, bevorzugt mindestens vier, mehr bevorzugt mindestens 5 und insbesondere mindestens sechs strahlungshärtbare Gruppen aufweist. Die Anzahl der strahlungshärtbaren Gruppen ist prinzipiell nach oben nicht beschränkt, jedoch können Polyurethan(meth)acrylate mit sehr hoher Anzahl an strahlungshärtbaren Gruppen unerwünschte Eigenschaften, wie beispielsweise hohe Viskosität oder schlechte Löslichkeit, aufweisen. Die hochfunktionellen Polyurethan(meth)acrylate (U) der vorliegenden Erfindung weisen zumeist nicht mehr als 100 strahlungshärtbare Gruppen, bevorzugt nicht mehr als 50, besonders bevorzugt nicht mehr als 30 und ganz besonders bevorzugt nicht mehr als 20 strahlungshärtbare Gruppen auf. Sie weisen im Mittel bevorzugt nicht weniger als 3,5, bevorzugt nicht weniger als 4,5, ganz besonders bevorzugt nicht weniger als 5 strahlungshärtbare Gruppen auf.

Die Polyurethan(meth)acrylate (U) haben ein Molekulargewicht M_{w} von mindestens 500, bevorzugt mindestens 600 und besonders bevorzugt 750 g/mol. Die obere Grenze des Molekulargewichts M_{w} ist bevorzugt 100.000 g/mol, besonders bevorzugt beträgt es nicht mehr als 80.000 und ganz besonders bevorzugt nicht mehr als 40.000 g/mol.

Die Angaben zur Polydispersität sowie zum zahlenmittleren und gewichtsmittleren Molekulargewicht Mₙ und M_{w} beziehen sich hier auf gelpermeationschromatographische Messungen, wobei Polymethylmethacrylat als Standard und Tetrahydrofuran oder Dimethylacetamid, je nachdem in welchem Lösungsmittel die Probe besser löslich ist, als Elutionsmittel verwendet wurde. Die Methode ist im Analytiker Taschenbuch Bd. 4, Seiten 433 bis 442 , Berlin 1984 beschrieben.

Die Polydispersität der Polyurethan(meth)acrylate (U) beträgt 1,1 bis 50, bevorzugt 1,2 bis 40, besonders bevorzugt 1,3 bis 30 und ganz besonders bevorzugt 1,5 bis 10.

Die Polyurethan(meth)acrylate (U) sind üblicherweise sehr gut löslich, d.h. man kann bei 25 °C klare Lösungen mit einem Gehalt bis zu 50 Gew.-%, in einigen Fällen sogar bis zu 80 Gew.-%, in Aceton, 2-Butanon, Tetrahydrofuran (THF), Ethylacetat, n-Butylacetat, Dimethylacetamid und zahlreichen anderen Lösemitteln darstellen, ohne dass mit bloßem Auge Gelpartikel detektierbar sind. Dies zeigt den geringen Vernetzungsgrad der Polyurethan(meth)acrylate.

Als Di- und Polyisocyanate (I) kommen die aus dem Stand der Technik bekannten aliphatischen, cycloaliphatischen und aromatischen Isocyanate in Frage, bevorzugt sind aliphatische und cycloaliphatische Isocyanate.

Als isocyanatgruppenhaltige Produkt (a) sind hier die Produkte (a1) und (a2) zusammenfassend bezeichnet und werden nach ihrer Funktionalität in die Produkte (a1) und (a2) unterteilt.

Diisocyanate (a1) sind solche Isocyanate, die eine Funktionalität von 2, d.h. zwei Isocyanatgruppen pro Molekül, aufweisen. Polyisocyanate (a2) sind solche Isocyanate, die im Mittel mehr als 2, bevorzugt im Mittel mindestens 3 NCO-Gruppen pro Molekül aufweisen.

Bevorzugte Di- oder Polyisocyanate (I) sind 2,4'- und 4,4'-Diphenylmethandiisocyanat (MDI), die Mischungen aus monomeren Diphenylmethandiisocyanaten und höherkernigen Homologen des Diphenylmethandiisocyanats (Polymer-MDI), Tetramethylendiisocyanat, Tetramethylendiisocyanat-Trimere, Hexamethylendiisocyanat, Hexamethylendiisocyanat-Trimere, Isophorondiisocyanat-Trimer, 2,4'- und 4,4'-Methylenbis(cyclohexyl)-diisocyanat, Xylylendiisocyanat, Tetramethylxylylendiisocyanat, Dodecyldiisocyanat, Lysinalkylester-diisocyanat, wobei Alkyl für C₁ bis C₁₀ steht, 2,2,4- oder 2,4,4-Tri-methyl-1,6-hexamethylendiisocyanat, 1,4-Diisocyanatocyclohexan, 1,3- oder 1,4-Bis-(isocyanatomethyl)cyclohexan oder 4-Isocyanatomethyl-1,8-octamethylendiisocyanat oder 3 (bzw. 4), 8 (bzw. 9)-Bis-(isocyanatomethyl)-tricyclo[5.2.1.0^{2.6}]decan-Isomeren-gemische.

Besonders bevorzugt sind Di- oder Polyisocyanate mit NCO-Gruppen unterschiedlicher Reaktivität, wie 2,4-Toluylendiisocyanat (2,4-TDI), 2,4'-Diphenylmethandiisocyanat (2,4'-MDI), Trüsocyanatotoluol, Isophorondiisocyanat (IPDI), 2-Butyl-2-ethylpentamethylendiisocyanat, 2-Isocyanatopropylcyclohexylisocyanat, 3(4)-Isocyanatomethyl-1-methylcyclohexyl-isocyanat, 1,4-Diisocyanato-4-methylpentan, 2,4'-Methylenbis-(cyclohexyl)diisocyanat und 4-Methyl-cyclohexan-1,3-diisocyanat (H-TDI).

Unterschiedliche Reaktivität im Sinne der vorliegenden Erfindung bedeutet einen Reaktivitätsunterschied zwischen den zu differenzierenden reaktiven Gruppen innerhalb des Moleküls unter den Reaktionsbedingungen, so daß der Quotient k₁/k₂ der Geschwindigkeitskoeffizienten k₁ und k₂ der jeweiligen reaktiven Gruppen für die betreffende Reaktion mindestens 1,25, bevorzugt mindestens 1,5, besonders bevorzugt mindestens 2, ganz besonders bevorzugt mindestens 2,5 und insbesondere mindestens 3 beträgt.

Weiterhin sind Isocyanate besonders bevorzugt, deren NCO-Gruppen zunächst gleich reaktiv sind, bei denen sich jedoch durch Erstaddition eines Alkohols, Mercaptans oder Amins an einer NCO-Gruppe ein Reaktivitätsabfall bei der zweiten NCO-Gruppe induzieren läßt. Beispiele dafür sind Isocyanate, deren NCO-Gruppen über ein delokalisiertes Elektronensystem gekoppelt sind, z.B. 1,3- und 1,4-Phenylendiisocyanat, 1,5-Naphthylendiisocyanat, Diphenyldiisocyanat, Tolidindiisocyanat oder 2,6-Toluylendiisocyanat.

Als Di- und Polyisocyanate (II) kommen alle aus dem Stand der Technik bekannten aliphatischen, cycloaliphatischen und aromatischen Isocyanate in Frage. Neben den oben genannten Di- und Polyisocyanaten (I) können weiterhin beispielsweise Oligo- oder Polyisocyanate verwendet werden, die sich aus den genannten Di- oder Triisocyanaten oder deren Mischungen durch Verknüpfung mittels Urethan-, Allophanat-, Harnstoff-, Biuret-, Uretdion-, Amid-, Isocyanurat-, Carbodiimid-, Uretonimin-, Oxadiazintrion- oder Iminooxadiazindion-Strukturen herstellen lassen.

Bei dem in der erfindungsgemäßen Reaktion eingesetzten Di- oder Polyisocyanat (II) kann es sich bevorzugt auch um ein anderes als das in dem Schritt (i) eingesetzten Di- oder Polyisocyanat (I) handeln.

In einer bevorzugten Ausführungsform handelt es sich bei der Verbindung (I) um ein Diisocyanat (a1) mit einer Funktionalität von 2 und bei der Verbindung (II) um ein Isocyanat mit einer Funktionalität von mehr als 2, bevorzugt mindestens 2,5, besonders bevorzugt mindestens 2,8 und ganz besonders bevorzugt mindestens 3.

Als Di- und Polyisocyanate (II) werden besonders bevorzugt verwendet 2,4'- und 4,4'-Diphenylmethandiisocyanat, Mischungen aus Diphenylmethandiisocyanaten und höherkernigen Homologen des Diphenylmethandiisocyanats (Polymer-MDI), 1,3- und 1,4-Phenylendiisocyanat, 4-Isocyanatomethyl-1,8-octamethylendiisocyanat, Hexamethylendiisocyanat, Isocyanurat-, Uretdion-, Urethan-, Allophanat-, Iminooxadiazindion- oder Biuretgruppen aufweisende Oligomere aus Hexamethylendiisocyanat oder Isophorondiisocyanat (IPDI), Urethan-, Allophanat-, Carbodiimid- oder Uretonimingruppen aufweisende Oligomere aus MDI oder Urethan-, Allophanat-, Carbodiimid- oder Uretonimingruppen aufweisende Oligomere aus TDI.

Sowohl für die Di- und Polyisocyanate (I) als auch für die Di- und Polyisocyanate (II) können auch Mischungen der genannten Isocyanate verwendet werden.

Als Monoisocyanate kommen z.B. Phenylisocyanat, o-, m- oder p-Tolylisocyanat, Naphthylisocyanat, Phenylsulfonylisocyanat, Toluolsulfonylisocyanat, Butylisocyanat, Hexylisocyanat, Cyclohexylisocyanat, Dodecylisocyanat oder Stearylisocyanat in Betracht. Bevorzugt werden Phenylisocyanat, Toluolsulfonylisocyanat, Cyclohexylisocyanat oder Stearylisocyanat verwendet.

Es spielt für die Isocyanate keine wesentliche Rolle, auf welchem Wege die Monoisocyanate, Di- und Polyisocyanate (I) oder (II) hergestellt worden sind, d.h. ob sie über ein Phosgenierungsverfahren oder ein phosgenfreies Verfahren erhalten worden sind.

Als Produkte (b) mit gegenüber Isocyanatgruppen reaktiven Gruppen sind hier die Produkte (b1) und (b2) zusammenfassend bezeichnet und werden nach ihrer Funktionalität in die Produkte (b1) und (b2) unterteilt.

Die bei der Herstellung des Additionsproduktes (A) verwendeten Verbindungen (b1) mit mindestens drei mit Isocyanat reaktiven Gruppen und/oder Verbindungen (b2) mit zwei mit Isocyanat reaktiven Gruppen weisen als mit Isocyanat reaktive Gruppen Hydroxygruppen, Mercaptogruppen und/oder Aminogruppen auf. Bevorzugt sind Hydroxy- und/oder Aminogruppen und besonders bevorzugt Hydroxygruppen.

In einer bevorzugten Ausführungsform enthalten die Verbindungen (b1) mit mindestens drei mit Isocyanat reaktiven Gruppen bevorzugt 3 - 6, besonders bevorzugt 3 - 5, ganz besonders bevorzugt drei oder vier mit Isocyanat reaktive Gruppen.

Ebenfalls können zur Herstellung des Additionsprodukts (A) Verbindungen (b1) mit mindestens drei mit Isocyanat reaktiven Gruppen und/oder Verbindungen (b2) mit zwei mit Isocyanat reaktiven Gruppen verwendet werden, die aus den oben genannten funktionellen Gruppen oder Gemischen davon ausgewählt sind und deren funktionelle Gruppen gegenüber NCO-Gruppen eine unterschiedliche Reaktivität aufweisen. Bevorzugt sind dabei Verbindungen mit mindestens einer primären und mindestens einer sekundären oder tertiären Hydroxygruppe, mindestens einer Hydroxygruppe und mindestens einer Mercaptogruppe oder mindestens einer Hydroxygruppe und mindestens einer Aminogruppe im Molekül, da die Reaktivität der Aminogruppe gegenüber der Hydroxygruppe bei der Umsetzung mit Isocyanat in der Regel deutlich höher ist.

Weiterhin bevorzugt sind mit Isocyanat reaktive Verbindungen, deren mit Isocyanat reaktive funktionellen Gruppen zunächst gleich reaktiv sind, bei denen sich jedoch durch Addition mindestens eines Isocyanates ein Reaktivitätsabfall, bedingt durch sterische oder elektronische Einflüsse, bei den restlichen mit Isocyanat reaktiven Gruppen induzieren läßt. Dies ist beispielsweise bei der Verwendung von Trimethylolpropan oder Pentaerythrit als Komponente (b1) der Fall.

Beispiele für (b1) Verbindungen mit mindestens drei mit Isocyanat reaktiven Gruppen sind Glycerin, Trimethylolmethan, Trimethylolethan, Trimethylolpropan, 1,2,4-Butantriol, 1,2,5-Pentantriol, 1,2,6-Hexantriol, 1,2,7-Heptantriol, 1,2,8-Oktantriol, 1,2,9-Nonantriol, 1,2,10-Decantriol, Tris(2-hydroxyethyl)isocyanurat, Tris(hydroxymethyl)-aminomethan, Tris(hydroxyethyl)aminomethan, 2-Amino-1,3-propandiol, 2-Amino-2-methyl-1,3-propandiol, Diethanolamin, Dipropanolamin, Diisopropanolamin, Ethanolpropanolamin, Bis(aminoethyl)amin, Bis(aminopropyl)amin, Tris(aminoethyl)amin, Tris(aminopropyl)amin, Trisaminononan, Tris-(2-hydroxyethyl) isocyanurat, Pentaerythrit, Dipentaerythrit, Bis(trimethylolpropan), Zuckeralkohole, wie z.B. Sorbit, Mannit, Diglycerol, Threit, Erythrit, Adonit (Ribit), Arabit (Lyxit), Xylit, Dulcit (Galactit), Maltit, Isomaltit, oder Zucker, wie zum Beispiel Glucose, tri- oder höherfunktionelle Polyetherole auf Basis von tri- oder höherfunktionellen Startermolekülen und Ethylenoxid und/oder Propylenoxid und/oder Butylenoxid, oder deren aminogruppenterminierte Derivate, die allgemein bekannt sind als Jeffamine®, oder tri- oder höherfunktionelle Polyesterole. Dabei sind Glycerin, Trimethylolethan, Trimethylolpropan, 1,2,4-Butantriol, 1,2,6-Hexantriol, Pentaerythrit, Polyetherole auf Basis von Glycerin, Trimethylolpropan oder Pentaerythrit, Diethanolamin, Dipropanolamin und Tris(hydroxymethyl)amino-methan besonders bevorzugt.

Beispiele für (b2) Verbindungen mit zwei mit Isocyanat reaktiven Gruppen sind Ethylenglykol, Diethylenglykol, Triethylenglykol, 1,2- und 1,3-Propandiol, Dipropylenglykol, Tripropylenglykol, Neopentylglykol, 1,2-, 1,3- und 1,4-Butandiol, 1,2-, 1,3- und 1,5-Pentandiol, 1,6-Hexandiol, Hydroxypivalinsäureneopentylglykolester, Propan-1,2-dithiol, Butan-1,2-dithiol, Mercaptoethanol, Mercaptopropanol, Mercaptobutanol, Ethylendiamin, Toluylendiamin, Isophorondiamin, Cysteamin, Ethanolamin, N-Methylethanolamin, 1,2- oder 1,3-Propanolamin, Isopropanolamin, 2-(Butylamino)ethanol, 2-(Cyclohexylamino)ethanol, 2-Amino-1-butanol, 2-(2'-Aminoethoxy)ethanol oder höhere Alkoxylierungsprodukte des Ammoniaks, 4-Hydroxypiperidin, 1-Hydroxyethylpiperazin, Aminopropanthiol oder difunktionelle Polyether- oder Polyesterole, sowie difunktionelle Polyetheramine, allgemein bekannt als Jeffamine®. Dabei sind Ethylenglykol,1,2- und 1,3-Propandiol, 1,2-, 1,3-und 1,4-Butandiol, Ethanolamin, 1,2-Propanolamin, Mercaptoethanol, 4-Hydroxypiperidin und 1-Hydroxyethylpiperazin oder Polyetherole besonders bevorzugt.

Bei den hier genannten Jeffaminen® der Firma Huntsman handelt es sich um Mono-, Di- oder Triamine, die auf der Basis von Polyethylenoxiden, Polypropylenoxiden oder gemischten Polyethylenoxiden/Polypropylenoxiden beruhen, und eine Molmasse bis zu ca. 5000 g/mol aufweisen können.

Beispiele für derartige Monoamine sind die sogenannten Jeffamine® M-Serien, die methylgekappte Polyalkylenoxide mit eine Aminofunktion darstellen, wie M-600 (XTJ-505), mit einem Propylenoxid (PO)/Ethylenoxid (EO)-Verhältnis von ca. 9:1 und einer Molmasse von ca. 600, M-1000 (XTJ-506): PO/EO-Verhältnis 3:19, Molmasse ca. 1000, M-2005 (XTJ-507): PO/EO-Verhältnis 29:6, Molmasse ca. 2000 oder M-2070: PO/EO-Verhältnis 10:31, Molmasse ca. 2000.

Beispiele für derartige Diamine sind die sogenannten Jeffamine® D- oder ED-Serien. Die D-Serie sind aminofunktionalisierte Polypropylendiole aus 3 - 4 1,2-Propyleneinheiten (Jeffamine® D-230, mittlere Molmasse 230), 6 - 7 1,2-Propyleneinheiten (Jeffamine® D-400, mittlere Molmasse 400), im Schnitt ca. 34 1,2-Propyleneinheiten (Jeffamine® D-2000, mittlere Molmasse 2000) oder im Schnitt ca. 69 1,2-Propyleneinheiten (Jeffamine® XTJ-510 (D-4000), mittlere Molmasse 4000). Diese Produkte können teilweise auch als Aminoalkohole vorliegen. Die ED-Serie sind Diamine auf Basis von Polyethylenoxiden, die idealiserterweise beidseitig propoxyliert sind, beispielsweise Jeffamine® HK-511 (XTJ-511) aus 2 Ethylenoxid- und 2 Propylenoxideinheiten mit einer mittleren Molmasse von 220, Jeffamine® XTJ-500 (ED-600) aus 9 Ethylenoxid- und 3,6 Propylenoxideinheiten mit einer mittleren Molmasse von 600 sowie Jeffamine® XTJ-502 (ED-2003) aus 38,7 Ethylenoxid- und 6 Propylenoxideinheiten mit einer mittleren Molmasse von 2000.

Beispiele für Triamine sind Jeffamine® T-403, ein Triamin auf Basis eines mit 5 - 6 1,2-Propyleneinheiten modifizierten Trimethylolpropan, Jeffamine® T-5000, ein Triamin auf Basis eines mit ca. 85 1,2-Propyleneinheiten modifizierten Glycerin sowie Jeffamine® XTJ-509 (T-3000), ein Triamin auf Basis eines mit 50 1,2-Propyleneinheiten modifizierten Glycerin.

Weiterhin sind auch Mischungen der genannten Verbindungen einsetzbar.

Bei der Herstellung des Additionsprodukts (A) ist es notwendig, das Verhältnis von Di-(a1) und/oder Polyisocyanat (a2) zu Verbindungen (b1) mit mindestens drei mit Isocyanat reaktiven Gruppen oder (b2) Verbindungen mit zwei mit Isocyanat reaktiven Gruppen oder Gemischen aus (b1) und (b2) so einzustellen, dass das resultierende Additionsprodukt (A) Isocyanatgruppen enthalten kann und im Mittel mindestens eine mit Isocyanat reaktive Gruppe enthält.

Beispielsweise liegt bei der Herstellung des Additionsproduktes (A) aus einem Diisocyanat (a1) und einem dreiwertigen Alkohol (b1) das Umsetzungsverhältnis bei 2 : 1, veranschaulicht durch die allgemeine Formel 1, 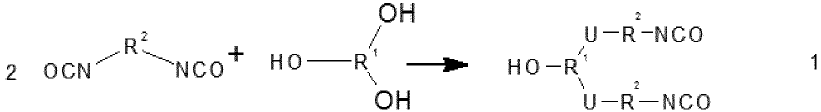 und bei der Herstellung des Additionsproduktes (A) aus einem Diisocyanat (a1) und einem vierwertigen Alkohol als (b1) das Umsetzungsverhältnis bei 3 : 1, schematisch veranschaulicht durch die allgemeine Formel 2, 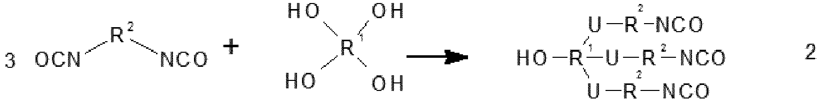 wobei in den Formeln 1 und 2 R¹ und R² einen organischen Rest und U eine Urethangruppe bedeuten.

Weiterhin kann die Herstellung des Additionsprodukts (A) zum Beispiel auch aus einem Triisocyanat (a2) und einer zweiwertigen, mit Isocyanat reaktiven Komponente (b2), veranschaulicht durch die allgemeine Formel 3 erfolgen, wobei das Umsetzungsverhältnis bei molar 1:1 liegt, R¹ und R² die gleiche Bedeutung wie in den Formeln 1 und 2 haben und Y zum Beispiel eine Harnstoffgruppe bedeutet.

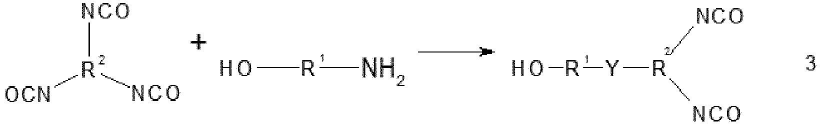

Werden zur Komponente (b1) zusätzlich Verbindungen (b2) mit zwei mit Isocyanat reaktiven Gruppen zugegeben, so bewirkt dies in der Regel eine Verlängerung der Ketten. Wie beispielsweise in der allgemeinen Formel 4 veranschaulicht, muss für jedes Mol der Komponente (b2) ein weiteres Mol an Di- oder Polyisocyanat (a1) oder (a2) (I) zugegeben werden.

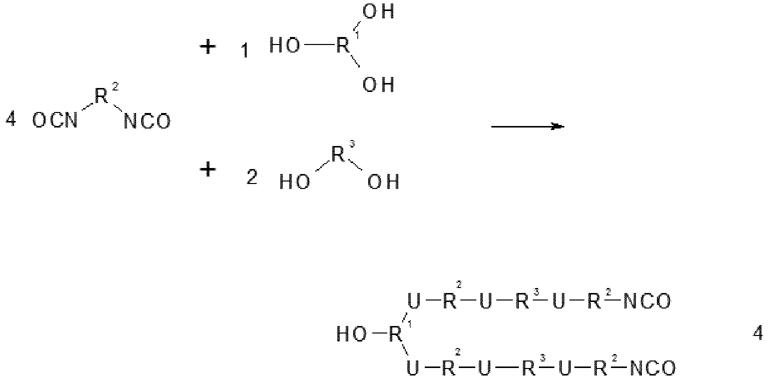

In Formel 4 bedeutet R³ einen organischen Rest, R¹, R² und U sind wie vorstehend beschrieben definiert.

Die Umsetzung ausschließlich difunktioneller Komponenten, also Verbindungen (a1) und (b2), führt zu einem linearen Produkt (A). Verzweigte Produkte können in der Folge nur dann erhalten werden, wenn diese in einem späteren Reaktionsschritt mit einer mehr als difunktionellen Verbindung umgesetzt werden, die gegenüber dem Additionsprodukt (A) reaktive Gruppen aufweist.

Zu mehr als difunktionellen Produkten gelangt man also, wenn mindestens eines der isocyanatgruppenhaltigen Produkte (a) und der mit Isocyanat reaktiven Gruppen enthaltenden Produkte (b) mehr als difunktionell ist, also im statistischen Mittel eine Funktionalität von mehr als 2 aufweist, beispielsweise mit den Kombinationen (a1) + (b2) oder (a2) + (b1) aber auch (a2) + (b2), (a1) + (a2) + (b1), (a1) + (a2) + (b2), (a2) + (b1) + (b2) sowie (a1) + (a2) + (b1) + (b2).

Die Umsetzung zum Additionsprodukt (A) erfolgt üblicherweise bei einer Temperatur von -20 bis 120°C, bevorzugt bei -10 bis 100°C. In einer bevorzugten Ausführungsform wird das Di- (a1) und/oder Polyisocyanat (a2) vorgelegt und die Komponenten (b1) und/oder (b2) oder das Gemisch aus (b1) und (b2) zugegeben. Die Additionsprodukte (A) sind oft nicht über einen längeren Zeitraum stabil und werden daher, falls gewünscht, bevorzugt direkt mit dem Di- oder Polyisocyanat (II) umgesetzt (Reaktionsschritt (iii)).

In einer bevorzugten Ausführungsform kann das Additionsprodukt (A) durch eine intermolekulare Additionsreaktion des Additionsproduktes (A) in ein Polyadditionsprodukt (P) überführt werden (Reaktionsschritt (ii)). Hierbei addiert sich eine mit Isocyanat reaktive Gruppe des Additionsproduktes (A), soweit vorhanden, an eine der Isocyanatgruppen eines weiteren Additionsproduktes (A), besonders bevorzugt reagiert eine Hydroxy- und/oder Aminogruppe mit einer Isocyanatgruppe unter Ausbildung einer Urethan- bzw. Harnstoffgruppe. Die Anzahl der Additionsprodukte (A), die sich zu einem Polyadditionsprodukt (P) addieren, ist im allgemeinen nicht beschränkt. Aus praktischen Gesichtspunkten wird üblicherweise die Additionsreaktion abgebrochen, bevor das Polyadditionsprodukt (P), z.B. aufgrund eines zu hohen Molekulargewichts oder aus sterischen Gründen, nachteilige Eigenschaften, wie beispielsweise eine zu hohe Viskosität oder eine zu schlechte Löslichkeit, aufweist. Daher bricht man die Reaktion ab, sobald das gewünschte Molekulargewicht erreicht ist, spätestens sobald die oben angegebenen Molekulargewichte Mw erreicht sind.

Aufgrund der Beschaffenheit der Additionsprodukte (A) ist es möglich, daß aus der Additionsreaktion verschiedene Polyadditionsprodukte (P) resultieren können, die Verzweigungen aber im wesentlichen keine Vernetzungen aufweisen. Ferner weisen die Polyadditionsprodukte (P) mehr als zwei Isocyanatgruppen auf und können eine oder mehrere mit Isocyanat reaktive Gruppen aufweisen. Die Anzahl der Isocyanatgruppen ergibt sich dabei aus der Beschaffenheit der eingesetzten Additionsprodukte (A) und dem Polyadditionsgrad.

Beispielsweise kann ein Additionsprodukt (A) gemäß der allgemeinen Formel 1 durch dreifache intermolekulare Addition zu zwei verschiedenen Polyadditionsprodukten (P), die in den allgemeinen Formeln 5 und 6 wiedergegeben werden, reagieren.

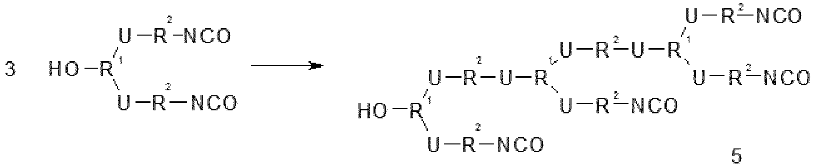

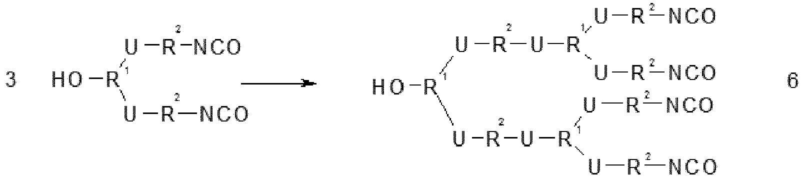

In Formel 5 und 6 sind R¹, R² und U wie vorstehend definiert.

Die intermolekulare Polyadditionsreaktion eines Additionsproduktes (A) zu einem Polyadditionsprodukt (P) kann üblicherweise und bevorzugt in situ nach Beendigung der Reaktion zu dem Additionsprodukt (A) durch eine Temperaturerhöhung durchgeführt werden, wenn das Additionsprodukt mindestens eine, bevorzugt genau eine gegenüber Isocyanat reaktive Gruppe aufweist.

Bedingt dadurch, daß die reaktiveren Gruppen aus den Verbindungen (a) und (b) in Schritt (i) im wesentlichen abreagiert sind, verbleiben im Reaktionsgemisch die unreaktiveren Gruppen. Diese erfordern für eine Weiterreaktion zu (P) eine erhöhte Reaktionstemperatur.

Ferner ist es auch möglich, sowohl durch Zugabe eines geeigneten Katalysators, als auch durch Wahl einer geeigneten Temperatur die intermolekulare Polyadditionsreaktion zu steuern.

Die Umsetzung wird, falls gewünscht, durch Zugabe eines geeigneten Katalysators beschleunigt. Solche Katalysatoren sind literaturbekannt, beispielsweise aus G. Oertel (Hrsg.), Polyurethane, 3. Auflage 1993, Carl Hanser Verlag, München - Wien, Seiten 104 bis 110, Kapitel 3.4.1. "Katalysatoren", bevorzugt sind organische Amine, insbesondere tertiäre aliphatische, cycloaliphatische oder aromatische Amine, Brønsted-Säuren und/oder Lewis-saure Organometallverbindungen, besonders bevorzugt sind Lewis-saure Organometallverbindungen. Bevorzugt handelt es sich dabei um Lewis-saure organische Metallverbindungen, für die z.B. Zinnverbindungen in Frage kommen, wie beispielsweise Zinn-(II)-salze von organischen Carbonsäuren, z.B. Zinn-(II)-diacetat, Zinn-(II)-dioctoat, Zinn-(II)-bis(ethylhexanoat) und Zinn-(II)-dilaurat und die Dialkylzinn-(IV)-salze von organischen Carbonsäuren, z.B. Dimethylzinn-diacetat, Dibutylzinn-diacetat, Dibutylzinn-dibutyrat, Dibutylzinn-bis(2-ethylhexanoat), Dibutylzinn-dilaurat, Dibutylzinn-maleat, Dioctylzinn-dilaurat und Dioctylzinn-diacetat. Zudem können Zink-(II)-Salze eingesetzt werden, wie beispielsweise Zink-(II)-dioctoat.

Auch Metallkomplexe wie Acetylacetonate des Eisens, Titans, Aluminiums, Zirkons, Mangans, Nickels, Zinks und Cobalts sind möglich.

Weitere Metallkatalysatoren werden von Blank et al. in Progress in Organic Coatings, 1999, Vol. 35, Seiten 19-29 beschrieben.

Als Zinn- und Zink-freie Alternativen werden u.a. Zirkonium, Wismut und AluminiumVerbindungen eingesetzt. Dies sind z.B. Zirkoniumtetraacetylacetonat (z.B. K-KAT® 4205 der Firma King Industries); Zirkoniumdionate (z.B. K-KAT® XC-9213; XC-A 209 und XC-6212 der Firma King Industries); Wismut-Verbindungen, insbesondere Tricarboxylate (z.B. K-KAT® 348, XC-B221; XC-C227, XC 8203 der Firma King Industries); Aluminiumdionat (z.B. K-KAT® 5218 der Firma King Industries). Zinn- und Zink-freie Katalysatoren werden ansonsten z.B. auch unter dem Handelsnamen Borchi® Kat der Firma Borchers, TK der Firma Goldschmidt oder BICAT® der Firma Shepherd, Lausanne angeboten.

Diese Katalysatoren sind für lösungsmittel-, wasser-basierte und/oder blockierte Systeme geeignet.

Molybdän-, Wofram- und Vanadium-Katalysatoren, werden insbesondere für den Umsatz blockierter Polyisocyanate unter WO 2004/076519 und WO 2004/076520 beschrieben.

Auch Cäsiumsalze können als Katalysatoren eingesetzt werden. Als Cäsiumsalze kommen dabei solche Verbindungen in Betracht, in denen folgende Anionen eingesetzt werden: F-, Cl⁻, ClO⁻, CLO₃⁻, ClO₄⁻, Br⁻, J-, JO₃⁻, CN⁻, OCN⁻, NO₂⁻ NO₃⁻, HCO₃⁻, CO₃²⁻, S²⁻, SH-, HSO₃⁻, SO₃²⁻, HSO₄⁻, SO₄²⁻, ,S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₙH₂ₙ₊₁)⁻, (CₙH₂ₙ₋₁O₂)⁻, (CₙH₂ₙ₋₃O₂)⁻ sowie (Cₙ₊₁H₂ₙ₋₂O₄)²⁻, wobei n für die Zahlen 1 bis 20 steht.

Bevorzugt sind dabei Cäsiumcarboxylate, bei denen das Anion den Formeln (CₙH₂ₙ₋₁O₂)⁻ sowie (Cₙ₊₁H₂ₙ₋₂O₄)²⁻ mit n gleich 1 bis 20, gehorcht. Besonders bevorzugte Cäsiumsalze weisen als Anionen Monocarboxylate der allgemeinen Formel (CₙH₂ₙ-₁O₂)⁻ auf, wobei n für die Zahlen 1 bis 20 steht. Hierbei sind insbesondere zu erwähnen Formiat, Acetat, Propionat, Hexanoat und 2-Ethylhexanoat.

Bevorzugte Lewis-saure organische Metallverbindungen sind Dimethylzinn-diacetat, Dibutylzinn-dibutyrat, Dibutylzinn-bis(2-ethylhexanoat), Dibutylzinn-dilaurat, Dioctylzinn-dilaurat, Zink-(II)-dioctoat, Zirkon-Acetylacetonat und Zirkon-2,2,6,6-tetramethyl-3,5-heptandionat.

Besonders bevorzugt ist jedoch Dibutylzinndilaurat.

Es ist auch denkbar, die Reaktion ohne Katalysator durchzuführen, in diesem Fall muß das Reaktionsgemisch jedoch höheren Temperaturen und/oder längeren Reaktionszeiten ausgesetzt werden.

Weiterhin ist auch denkbar, die Reaktion bei höherer Temperatur als oben angegeben durchzuführen, wobei die Schritte (i) und (ii) parallel ablaufen. Aufgrund der geringeren Selektivität bei der Reaktion der reaktiven Gruppen und der damit verminderten Steuerungsmöglichkeit der Molekülarchitektur ist diese Variante jedoch weniger bevorzugt Zum Abbruch der intermolekularen Polyadditionsreaktion gibt es verschiedene Möglichkeiten. Beispielsweise kann die Temperatur auf einen Bereich abgesenkt werden, in dem die Additionsreaktion zum Stillstand kommt und das Additionsprodukt (A) oder das Polyadditionsprodukt (P) lagerstabil ist.

In einer bevorzugten Ausführungsform wird, sobald aufgrund der intermolekularen Additionsreaktion des Additionsproduktes (A) ein Polyadditionsprodukt (P) mit gewünschten Polyadditionsgrad vorliegt, dem Polyadditionsprodukt (P) zum Abbruch der Polyadditionsreaktion ein Monoisocyanat oder besonders bevorzugt ein Di- oder Polyisocyanat (II) zugegeben (Schritt (iii)). Durch Umsetzung des Polyadditionsprodukts (P) mit dem Monoisocyanat oder dem Di- oder Polyisocyanat (II) erhält man hochfunktionelle Polyisocyanat als Ausgangsprodukt für den Schritt (iv).

Wird beispielsweise mit einem Polyadditionsprodukt (P) der allgemeinen Formel 5 ein Diisocyanat (II) im Verhältnis (P) : (II) = 2 :1 umgesetzt, so ist ein hochfunktionelles Polyisocyanat der allgemeinen Formel 7 erhältlich.

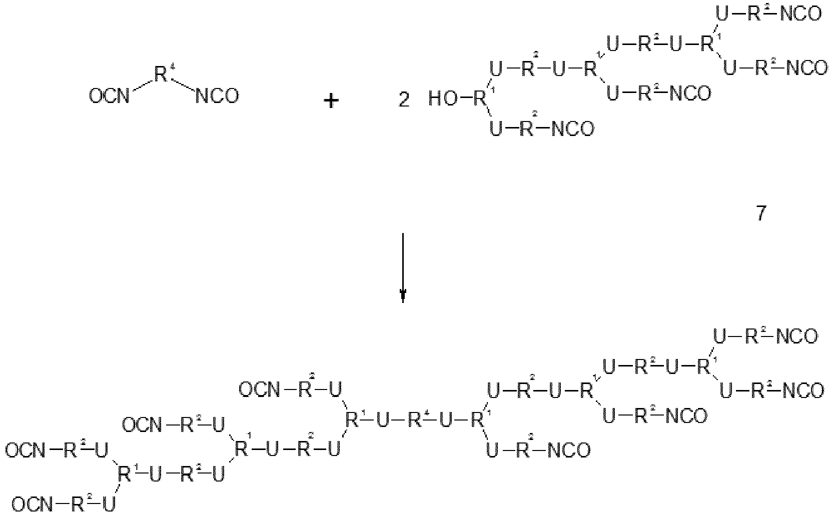

In Formel 7 sind R¹, R² und U wie vorstehend definiert und R⁴ bedeutet einen organischen Rest, der vorzugsweise nicht mit R² identisch ist.

Alternativ kann das Di- oder Polyisocyanat (II) auch zu einem Additionsprodukt (A) gegeben werden, das noch nicht in einer intermolekularen Additionsreaktion zu einem Polyadditionsprodukt (P) umgesetzt wurde.

Es ist jedoch zumeist technisch vorteilhaft, die intermolekulare Additionsreaktion zumindest in geringen Umfang durchzuführen, da gegebenenfalls im Additionsprodukt (A) noch geringe Mengen Di- oder Polyisocyanat (I) als Verunreinigung enthalten sein können und diese Verunreinigungen dann durch die intermolekulare Polyadditionsreaktion in das Polyadditionsprodukt (P) mit eingebaut werden können.

Die nach dem beschriebenen Verfahren hergestellten Polyisocyanate können in an sich bekannter Weise, beispielsweise durch Dünnschichtdestillation bei einer Temperatur von 100 bis 180 °C, gegebenenfalls im Vakuum, gegebenenfalls zusätzlich mit Durchleiten von inertem Strippgas, oder Extraktion von gegebenenfalls vorhandenen Lösungs- oder Verdünnungsmittel und/oder vorzugsweise von überschüssigen, nichtumgesetzten bevorzugt (cyclo)aliphatischen Diisocyanaten (I) befreit werden, so daß die Polyisocyanate mit einem Gehalt an monomeren Diisocyanaten von z.B. unter 1,0 Gew.-%, vorzugsweise unter 0,5 Gew.-%, besonders bevorzugt unter 0,3, ganz besonders bevorzugt unter 0,2 und insbesondere nicht mehr als 0,1 Gew% erhältlich sind.

Bei der Umsetzung des Additionsproduktes (A) und/oder des Polyadditionsprodukts (P) mit dem Di- oder Polyisocyanat (II) wird üblicherweise mindestens eine Isocyanatgruppe des Di- oder Polyisocyanats (II) mit der mit Isocyanat reaktiven Gruppe des Additionsprodukts (A) oder des Polyadditionsprodukts (P) zur Reaktion gebracht. In einer bevorzugten Ausführungsform werden mindestens 10 %, insbesondere mindestens 40 % und besonders bevorzugt 50 - 100% der freien Isocyanatgruppen des Di- oder Polyisocyanats (II) mit einer entsprechenden Anzahl an Äquivalenten eines Additionsproduktes (A) und/oder Polyadditionsprodukts (P) zu dem hochfunktionellen Polyisocyanat umgesetzt.

In einer weiteren Ausführungsform wird zuerst eine Isocyanatgruppe eines Di- oder Polyisocyanats (II) mit einem Additionsprodukt (A1) oder einem Polyadditionsprodukt (P1) umgesetzt, anschließend wird mindestens eine weitere Isocyanatgruppe des Di- oder Polyisocyanats (II) mit einem Additionsprodukt (A2) oder einem Polyadditionsprodukt (P2) umgesetzt, wobei die Additionsprodukte (A1) und (A2) bzw. die Polyadditionsprodukte (P1) und (P2) nicht identisch sind. Für diese Ausführungsform wird vorzugsweise ein Di- oder Polyisocyanat (II) verwendet, das Isocyanatgruppen mit unterschiedlicher Reaktivität gegenüber den mit Isocyanat reaktiven Gruppen der Komponenten (A) und/oder (P) aufweist.

Im Schritt (iv) wird das Additionsprodukt (A) und/oder das Polyadditionsprodukt (P) aus einem der vorstehenden Schritte mit einer strahlungshärtbaren Verbindung (c) umgesetzt.

Verbindungen (c) weisen mindestens eine, bevorzugt genau eine gegenüber Isocyanat reaktive Gruppe und mindestens eine Meth(acrylat)gruppe, beispielsweise ein bis vier, bevorzugt ein bis drei, besonders bevorzugt ein bis zwei und ganz besonders bevorzugt genau eine Meth(acrylat)gruppe auf.

Bevorzugt weisen die Komponenten (c) ein Molgewicht unter 1000 g/mol auf, besonders bevorzugt unter 700 g/mol, ganz besonders bevorzugt unter 500 g/mol und insbesondere unter 300 g/mol. Spezielle Verbindungen (c) weisen ein Molgewicht unter 250 oder sogar unter 200 g/mol auf.

Gegenüber Isocyanat reaktive Gruppen können z.B. sein -OH, -SH, -NH₂ und -NHR⁵, wobei R⁵ Wasserstoff oder eine 1 bis 4 Kohlenstoffatome enthaltende Alkylgruppe, wie z.B. Methyl, Ethyl, n-Propyl, *iso*-Propyl, n-Butyl, *iso-Butyl, sek-*Butyl oder *tert-*Butyl*,* bedeutet.

Komponenten (c) können z.B. Monoester von α,β-ungesättigten Carbonsäuren, wie Acrylsäure, Methacrylsäure, Crotonsäure, Itaconsäure, Fumarsäure, Maleinsäure, Acrylamidoglykolsäure, Methacrylamidoglykolsäure, bevorzugt sind Acrylsäure und Methacrylsäure, oder Vinylether mit Di- oder Polyolen sein, die vorzugsweise 2 bis 20 C-Atome und wenigstens zwei Hydroxygruppen aufweisen, wie Ethylenglykol, Diethylenglykol, Triethylenglykol, 1,2-Propylenglykol, 1,3-Propylenglykol, 1,1-Dimethyl-1,2-Ethandiol, Dipropylenglykol, Triethylenglykol, Tetraethylenglykol, Pentaethylenglykol, Tripropylenglykol, 1,2-, 1,3- oder 1,4-Butandiol, 1,5-Pentandiol, Neopentylglykol, 1,6-Hexandiol, 2-Methyl-1,5-pentandiol, 2-Ethyl-1,4-butandiol, 1,4-Dimethylolcyclohexan, 2,2-Bis(4-hydroxycyclohexyl)propan, Glycerin, Trimethylolethan, Trimethylolpropan, Trimethylolbutan, Pentaerythrit, Ditrimethylolpropan, Erythrit, Sorbit, Poly-THF mit einem Molgewicht zwischen 162 und 2000, Poly-1,3-propandiol mit einem Molgewicht zwischen 134 und 400 oder Polyethylenglykol mit einem Molgewicht zwischen 238 und 458. Weiterhin können auch Ester oder Amide der (Meth)acrylsäure mit Aminoalkoholen z. B. 2-Aminoethanol, 2-(Methylamino)ethanol, 3-Amino-1-propanol, 1-Amino-2-propanol oder 2-(2-Aminoethoxy)ethanol, 2-Mercaptoethanol oder Polyaminoalkane, wie Ethylendiamin oder Diethylentriamin, oder Vinylessigsäure verwendet werden.

Beispiele für Amide ethylenisch ungesättigter Carbonsäuren mit Aminoalkoholen sind Hydroxyalkyl(meth)acrylamide wie N-Hydroxymethylacrylamid, N-Hydroxymethylmethacrylamid, N-Hydroxyethylacrylamid, N-Hydroyxethylmethacrylamid, 5-Hydroxy-3-oxapentyl(meth)acrylamid, N-Hydroxyalkylcrotonamide wie N-Hydroxymethylcrotonamid oder N-Hydroxyalkylmaleinimide wie N-Hydroxyethylmaleinimid.

Bevorzugt verwendet werden 2-Hydroxyethyl(meth)acrylat, 2- oder 3-Hydroxypropyl-(meth)acrylat, 1,4-Butandiolmono(meth)acrylat, Neopentylglykolmono(meth)acrylat, 1,5-Pentandiolmono(meth)acrylat, 1,6-Hexandiolmono(meth)acrylat, Glycerinmono- und di(meth)acrylat, Trimethylolpropanmono- und di(meth)acrylat, Pentaerythritmono-, -di- und -tri(meth)acrylat sowie 4-Hydroxybutylvinylether, 2-Aminoethyl(meth)acrylat, 2-Aminopropyl(meth)acrylat, 3-Aminopropyl(meth)acrylat, 4-Aminobutyl(meth)acrylat, 6-Aminohexyl(meth)acrylat, 2-Thioethyl(meth)acrylat, 2-Aminoethyl(meth)acrylamid, 2-Aminopropyl(meth)acrylamid, 3-Aminopropyl(meth)acrylamid, 2-Hydroxyethyl(meth)-acrylamid, 2-Hydroxypropyl(meth)acrylamid oder 3-Hydroxypropyl(meth)acrylamid. Besonders bevorzugt sind 2-Hydroxyethylacrylat, 2-Hydroxyethylmethacrylat, 2- oder 3-Hydroxypropylacrylat, 1,4-Butandiolmonoacrylat, 3-(Acryloyloxy)-2-hydroxypropyl-(meth)acrylat sowie die Monoacrylate von Polyethylenglykol der Molmasse von 106 bis 238.

In einer bevorzugten Ausführungsform ist die Komponente (c) ausgewählt aus der Gruppe bestehend aus 2-Hydroxyethylacrylat, 2-Hydroxyethylmethacrylat, 2- oder 3-Hydroxypropylacrylat, 2- oder 3-Hydroxypropylmethacrylat, 1,4-Butandiolmonoacrylat, 1,4-Butandiolmonomethacrylat, 1,2- oder 1,3-Diacrylat von Glycerin, Trimethylolpropandiacrylat, Pentaerythrittriacrylat, Ditrimethylolpropantriacrylat und Dipentaerythritpentaacrylat, bevorzugt aus 2-Hydroxyethylacrylat und 2-Hydroxyethylmethacrylat und besonders bevorzugt handelt es sich um 2-Hydroxyethylmethacrylat.

In einer beispielhaften Ausführungsform kann es sich bei der Komponente (c) um technische Gemische der Acrylierung oder Methacrylierung von Trimethylolpropan, Pentaerythrit, Ditrimethylolpropan oder Dipentaerythrit handeln. Dabei handelt es sich zumeist um Gemische vollständig und unvollständig (meth)acrylierter Polyole. Beispielhaft geeignet sind technische Gemische der Acrylierung von Pentaerythrit, die zumeist eine OH-Zahl gemäß DIN 53240 von 99 bis 115 mg KOH/g aufweisen und überwiegend aus Pentaerythrittriacrylat und Pentaerythrittetraacrylat bestehen, sowie untergeordnete Mengen von Pentaerythritdiacrylat enthalten können. Dies hat den Vorteil, daß Pentaerythrittetraacrylat nicht in das Polyurethan eingebaut wird, sondern gleichzeitig als Reaktivverdünner (Verbindung (V)) fungiert.

Um eine unerwünschte Polymerisierung der (Meth)acrylatgruppen während der Reaktion zu vermeiden, können Polymerisationsinhibitoren zugesetzt werden. Derartige Inhibitoren sind beispielsweise beschrieben in WO 03/035596, Seite 5, Zeile 35 bis Seite 10, Zeile 4, worauf hiermit im Rahmen der vorliegenden Offenbarung Bezug genommen sei.

Es kann eine bevorzugte Ausführungsform der vorliegenden Erfindung darstellen, einbaubare Polymerisationsinhibitoren, d.h. solche, die eine-OH oder -NH₂-Gruppe, d.h. eine gegenüber Isocyanat reaktive Gruppe, enthalten, einzusetzen. Ein bevorzugtes Beispiel dafür ist 4-Hydroxy-2,2,6,6-tetramethyl-piperidin-N-oxyl oder 4-Amino-2,2,6,6-tetramethyl-piperidin-N-oxyl.

Zur Durchführung des Schrittes (iv) werden die Ausgangskomponenten (A) und/oder (P) mit der Verbindung (c) bei Temperaturen von 40 bis 180°C, vorzugsweise 50 bis 150°C, unter Einhaltung eines NCO-/OH-Äquivalentverhältnisses von 1 : 0,5 bis 1:2, vorzugsweise von 1 :0,7 bis 1 : 1,5, besonders bevorzugt 1 : 0,9 bis 1 : 1,1 miteinander umgesetzt.

In einer bevorzugten Ausführungsform wird die Reaktion so durchgeführt, daß das Produkt am Ende des Reaktionsschrittes (iv) einen NCO-Gehalt von unter 2 Gew%, bevorzugt unter 1 Gew%, besonders bevorzugt unter 0,5 Gew%, ganz besonders bevorzugt unter 0,3 Gew% und insbesondere unter 0,1 Gew% aufweist.

Bevorzugt werden von den im Additions- (A) bzw. Polyadditionsprodukt (P) vorliegenden Isocyanatgruppen mindestens 80 % mit Verbindung (c) umgesetzt, besonders bevorzugt mindestens 85%, ganz besonders bevorzugt mindestens 90%, insbesondere mindestens 95 % und speziell 98 bis 100%.

Die Reaktionsdauer beträgt in der Regel 10 min bis 5 Stunden, bevorzugt 15 min bis 4 Stunden, besonders bevorzugt 20 bis 180 min und ganz besonders bevorzugt 30 bis 120 min.

Zur Beschleunigung der Reaktion können gegebenenfalls geeignete Katalysatoren eingesetzt werden. Dabei kann es sich um die gleichen handeln, wie oben aufgeführt.

Die Reihenfolge der Vermischung der Komponenten (A), (P) und Komponente (c) ist dabei erfindungsgemäß nicht wesentlich, beispielsweise können die Komponenten gleichmäßig miteinander vermischt werden, Komponente (c) zumindest teilweise vorgelegt und (A) und/oder (P) dazu hinzugegeben werden oder (A) oder (P) zumindest teilweise vorgelegt, Komponente (c) hinzugegeben und die letzte Komponente hinzugegeben werden.

Der Verlauf der Umsetzung kann durch z. B. titrimetrische Bestimmung des NCO-Gehaltes gemäß DIN 53185 verfolgt werden. Nach Erreichen des angestrebten NCO-Gehaltes wird die Reaktion abgebrochen. Dies kann bei rein thermischer Reaktionsführung beispielsweise durch Abkühlen des Reaktionsgemisches auf Raumtemperatur erfolgen. Bei der Verwendung eines Katalysators der genannten Art wird die Umsetzung im allgemeinen aber durch Zugabe geeigneter Desaktivatoren abgestoppt. Als Desaktivatoren eignen sich beispielsweise anorganische oder organische Säuren, die entsprechenden Säurehalogenide und Alkylierungsmittel. Beispielhaft genannt seien Phosphorsäure, Monochloressigsäure, Dodecylbenzolsulfonsäure, Benzoylchlorid, Dimethylsulfat und vorzugsweise Dibutylphosphat sowie Di-2-ethylhexylphosphat.
Die Desaktivierungsmittel können in Mengen von 1 bis 200 Mol-%, vorzugsweise 20 bis 100 Mol-%, bezogen auf die Mole an Katalysator, eingesetzt werden.

Um eine gute Kompatibilität der Verbindungen (U) und (V) zu erreichen ist die Verbindung (U) im wesentlichen frei von ionischen Gruppen oder in ionische Gruppen überführbaren Gruppen, wie beispielsweise Säuregruppen, insbesondere Carboxy-, Sulfon- und Phosphonsäuregruppen. Bevorzugt beträgt deren Anteil weniger als 100 mmol bsonders bevorzugt weniger als 50 mmol und speziell weniger als 40 mmol pro 100g Verbindung (U).

Bevorzugt weist die Verbindung (U) ferner einen Gehalt von hydrophilen Gruppen, wie bevorzugt Hydroxy- und/oder Amingruppen, unter 200 mmol besonders bevorzugt unter 100 mmol und speziell unter 80 mmol pro 100g Verbindung (U) und einen NCO-Gehalt von unter 0,5 Gew.% auf.

Bei der Verbindung (V) handelt es sich um mindestens eine, beispielsweise ein bis drei, bevorzugt ein bis zwei und besonders bevorzugt genau eine Verbindung (V), die mindestens eine, bevorzugt mindestens zwei radikalisch polymerisierbare, bevorzugt strahlungshärtbare Gruppen aufweist, beispielsweise zwei bis sechs, bevorzugt zwei bis vier und besonders bevorzugt zwei bis drei.

Bevorzugt weisen die Verbindungen (V) eine niedrige Viskosität auf, bevorzugt von weniger als 15000 mPas (bei 25 °C gemäß DIN EN ISO 3219/A.3).

Die Verbindungen (V) weisen ein mittleres Molekulargewicht bis zu 1000, bevorzugt bis zu 750 g/mol auf. Bevorzugt handelt es sich um ein Polyether(meth)acrylat oder um ein (Meth)acrylat eines Di-, Tri- oder Tetraols oder um ein Urethandi(meth)acrylat aus Basis eines Diisocyanats.

Besonders bevorzugte Verbindungen (V) weisen einen Siedepunkt von mehr als 200 °C bei Normaldruck auf.

Bei den Verbindungen (V) kann es sich beispielsweise um Reaktivverdünner handeln, wie sie allgemein in P.K.T. Oldring (Herausgeber), Chemistry & Technology of UV & EB Formulations for Coatings, Inks & Paints, Vol. II, Chapter III: Reactive Diluents for UV & EB Curable Formulations, Wiley and SITA Technology, London 1997 beschrieben sind.

Verbindungen (V) sind gegenüber den Reaktionspartnern aus Reaktionschritt (i) inert.

Unter dem Begriff "inert" wird dabei verstanden, daß während der Dauer des Reaktionsschrittes (i) weniger als 10 mol% der Verbindungen (V), bevorzugt weniger als 5 mol%, besonders bevorzugt weniger als 3 mol% mit den Reaktionspartnern aus Reaktionschritt (i) reagieren.

Beispiele für Verbindungen (V) mit einer radikalisch polymerisierbaren Gruppe sind Acrylsäuremethylester und Methacrylsäuremethylester.

Bevorzugte Verbindungen (V) sind die Diester und Polyester von (Meth)acrylsäure mit Diolen oder Polyolen. Besonders bevorzugt sind Hexandioldiacrylat, Hexandioldimethacrylat, Octandioldiacrylat, Octandioldimethacrylat, Nonandioldiacrylat, Nonandioldimethacrylat, Decandioldiacrylat, Decandioldimethacrylat, Pentaerythrittetraacrylat, Dipentaerythrittetraacrylat, Dipentaerythrittriacrylat, Pentaerythrittetraacrylat, etc. Bevorzugt sind auch die Ester alkoxylierter Polyole, mit α,β-ethylenisch ungesättigten Mono- und/oder Dicarbonsäuren wie z. B. die Polyacrylate oder -methacrylate von im statistischen Mittel drei- bis 20fach, bevorzugt drei- bis 15fach, besonders bevorzugt drei- bis neunfach alkoxyliertem, besonders ethoxyliertem Trimethylolpropan, Glycerin oder Pentaerythrit sowie von Diethylenglykol, Triethylenglykol, Dipropylenglykol oder Tripropylenglykol. Geeignet sind weiterhin die Ester alicylischer Diole, wie Cyclohexandioldi(meth)acrylat und Bis(hydroxymethyl)cyclohexandi(meth)acrylat.

Besonders bevorzugt ist die Verbindung (V) ausgewählt aus der Gruppe bestehend aus Ethylenglykoldi(meth)acrylat, Diethylenglykoldi(meth)acrylat, Triethylenglykoldi(meth)acrylat, Butandioldi(meth)acrylat, Hexandioldi(meth)acrylat, Trimethylolpropanmono-, di- oder tri(meth)acrylat, Pentaerythritmono-, di-, tri- oder tetra(meth)acrylat und 2,2-Bis-4-(2-hydroxy-3-methacryloxy) phenylpropan.

Es stellt eine weitere Ausführungsform der vorliegenden Erfindung dar, als Verbindungen (V) NCO-Gruppen freie Umsetzungsprodukte aus aliphatischen oder aromatischen Diisocyanaten und (Meth)Acrylsäureestern, die mit Isocyanat reaktive Gruppen, vorzugsweise OH-Gruppen, tragen, einzusetzen. Eingesetzt werden können zum Beispiel Reaktionsprodukte aus aliphatischen oder aromatischen Diisocyanaten und Hydroxyethyl(meth)acrylat, Hydroxypropyl(meth)acrylat oder Hydroxybutyl(meth)acrylat. Als Diisocyanate bevorzugt sind sind 2,4'- und 4,4'-Diphenylmethandiisocyanat (MDI), 2,4- oder 2,6-Toluylendiisocyanat (TDI), Tetramethylendiisocyanat, Hexamethylendiisocyanat, 2,4'- und 4,4'-Methylenbis(cyclo-hexyl)-diisocyanat, Xylylendiisocyanat, Tetramethylxylylendiisocyanat, Dodecyldiisocyanat, Lysinalkylester-diisocyanat, wobei Alkyl für C₁ bis C₁₀ steht, 2,2,4- oder 2,4,4-Tri-methyl-1,6-hexamethylendiisocyanat, 1,4-Diisocyanatocyclohexan, 1,3- oder 1,4-Bis-(isocyanatomethyl)cyclohexan, Triisocyanatotoluol, Isophorondiisocyanat (IPDI), 2-Butyl-2-ethylpentamethylendiisocyanat, 2-Isocyanatopropylcyclohexylisocyanat, 3(4)-Isocyanatomethyl-1-methylcyclohexylisocyanat, 1,4-Diisocyanato-4-methylpentan oder und 2- oder 4-Methyl-cyclohexan-1,3-diisocyanat (H-TDI) oder Gemische der vorgenannten Isocyanate.

Besonders bevorzugt sind Umsetzungsprodukte aus aliphatischen Diisocyanaten, wie Tetramethylendiisocyanat, Hexamethylendiisocyanat, 2,4'- und 4,4'-Methylenbis(cyclohexyl)-diisocyanat, Xylylendiisocyanat, Tetramethylxylylendiisocyanat, Dodecyldiisocyanat, Lysinalkylester-diisocyanat, wobei Alkyl für C₁ bis C₁₀ steht, 2,2,4- oder 2,4,4-Tri-methyl-1,6-hexamethylendiisocyanat, 1,4-Diisocyanatocyclohexan, 1,3- oder 1,4-Bis-(isocyanatomethyl)cyclohexan, Isophorondiisocyanat (IPDI), 2-Butyl-2-ethylpentamethylendiisocyanat, 2-Isocyanatopropylcyclohexylisocyanat, 3(4)-Isocyanatomethyl-1-methylcyclohexyl-isocyanat, 1,4-Diisocyanato-4-methylpentan oder und 2- oder 4-Methyl-cyclohexan-1,3-diisocyanat (H-TDI) und Hydroxyethyl(meth)acrylat, Hydroxypropyl(meth)acrylat oder Hydroxybutyl(meth)acrylat. Ganz besonders bevorzugt sind Umsetzungsprodukte aus Hexamethylendiisocyanat, Isophorondiisocyanat, 2,2,4- oder 2,4,4-Tri-methyl-1,6-hexamethylendiisocyanat und Hydroxyethylmethacrylat, Hydroxypropylmethacrylat oder Hydroxybutylmethacrylat sowie speziell das Umsetzungsprodukt aus 2,2,4- und/oder 2,4,4-Tri-methyl-1,6-hexamethylendiisocyanat und zwei Äquivalenten Hydroxyethylmethacrylat, bekannt unter dem Trivialnamen Urethandimethacrylat oder UDMA.

Das Mengenverhältnis zwischen den Verbindungen (U) und (V) ist wie folgt:
(U) 30 - 99 Gew%, bevorzugt 50 - 90, besonders bevorzugt 60 - 80 Gew% und
(V) 1 - 70 Gew%, bevorzugt 10 - 50 , besonders bevorzugt 20 - 40 Gew%.

Durch die erfindungsgemäße Reaktionsführung in Gegenwart eines Reaktivverdünners (V) kann auf die Anwesenheit eines Lösungsmittels verzichtet werden, weniger bevorzugt kann jedoch ein Lösungsmittel anwesend sein:
Dazu kann Verfahren das gegebenenfalls in einem geeigneten, gegenüber den reaktiven Gruppen der Reaktionspartner inerten Lösemittel durchgeführt werden. Geeignete Lösemittel sind beispielsweise die an sich bekannten üblichen Lacklösemittel, wie z. B. Ethylacetat, Butylacetat, Ethylenglykolmonomethyl- oder -ethyletheracetat, 1-Methoxypropyl-2-acetat, 3-Methoxy-n-butylacetat, Aceton, 2-Butanon, *iso*Butylmethylketon, 4-Methyl-2-pentanon, Cyclohexanon, Cyclopentanon, Toluol, Xylol, Chlorbenzol, Testbenzin, höher substituierte Aromaten, wie sie beispielsweise unter den Bezeichnungen Solventnaphtha®, Solvesso®, Shellsol®, Isopar®, Nappar® und Diasol® im Handel sind, Propylenglykoldiacetat, Diethylenglykoldimethylether, Dipropylenglykoldimethylether, Diethylenglykolethyl- und -butyletheracetat, N-Methylpyrrolidon und N-Methylcaprolactam, sowie bevorzugt Kohlensäureester oder Lactone, die in der EP-A1 697 424, S. 4, Z. 4 bis 32 genannt sind, besonders bevorzugt Dimethylcarbonat, Diethylcarbonat, 1,2-Ethylencarbonat und 1,2-Propylencarbonat, Lactone, wie β-Propiolacton, γ-Butyrolacton, ε-Caprolacton und ε-Methylcaprolacton, aber auch oder beliebige Gemische solcher Lösemittel.

Die erfindungsgemäß erhältlichen Mischungen können für den Einsatz in Lacken selbstverständlich mit üblichen Hilfs- und Zusatzmitteln der Lacktechnologie versetzt werden. Hierzu gehören beispielsweise Entschäumer, Verdicker, Verlaufshilfsmittel, Pigmente, Emulgatoren, Dispergierhilfsmittel und auch Lösemittel. Die gewünschte Verarbeitungsviskosität wird durch Zugabe von weiterem Reaktivverdünner (V) oder, weniger bevorzugt, mindestens einem der oben genannten Lösungsmittel eingestellt.

Die Beschichtungsmitteln können insbesondere in Grundierungen, Füllern, pigmentierten Decklacken und Klarlacken im Bereich Autoreparatur- oder Großfahrzeuglackierung und Flugzeugen eingesetzt werden. Besonders geeignet sind solche Beschichtungsmittel für Anwendungen, in denen eine besonders hohe Applikationssicherheit, Außenwitterungsbeständigkeit, Härte und Flexibilität gefordert werden, wie in der Autoreparatur- und Großfahrzeuglackierung.

Insbesondere werden Beschichtungsmassen, enthaltend die nach dem erfindungsgemäß erhältlichen Mischungen, als oder in Automobilklar- und -decklacke(n) eingesetzt. Weitere bevorzugte Einsatzgebiete sind Can-Coating und Coil-Coating.

Unter "Coil-Coating" versteht man das kontinuierliche Beschichten von Metallbändern mit meist flüssigen Beschichtungsstoffen. Gewalzte Metallbänder werden nach der Herstellung zum Lagern und Transportieren zu Rollen (sogenannten "coils") aufgewickelt. Diese Metallbänder stellen das Ausgangsmaterial für die meisten flächigen metallischen Werkstücke dar, beispielsweise Automobilteile, Karosserieteile, Geräteverkleidungen, Fassadenverkleidungen, Deckenverkleidungen oder Fensterprofile. Dazu werden die geeigneten Metallbleche mittels geeigneter Techniken wie Stanzen, Bohren, Falzen, Profilieren und/oder Tiefziehen ausgeformt. Größere Bauteile, wie beispielsweise Automobilkarosserien werden gegebenenfalls durch Verschweißen mehrerer Einzelteile zusammengefügt.

Zur Beschichtung werden 0,2 bis 2 mm dicke und bis zu 2 m breite Metallbänder mit einer Geschwindigkeit von bis zu 200 m/min durch eine coil-coating-Anlage transportiert und dabei beschichtet. Hierzu können beispielsweise kaltgewalzte Bänder aus weichen Stählen oder Baustählen, elektrolytisch verzinktes Feinblech, feuerverzinktes Stahlband oder Bänder aus Aluminium bzw. Aluminiumlegierungen eingesetzt werden. Typische Anlagen umfassen eine Aufgabestation, einen Bandspeicher, eine Reinigungs- und Vorbehandlungszone, eine erste Lackierstation nebst Einbrennofen und folgender Kühlzone, eine zweite Lackierstation mit Ofen, Kaschierstation und Kühlung sowie einen Bandspeicher und Aufwickler.

Charakteristisch für coil-coatings sind dünne Schichten der Beschichtungsmassen, die eine Trockenschichtdicke von zumeist deutlich unter 80 µm, oftmals unter 60 µm, unter 50 µm und sogar unter 40 µm aufweisen. Zudem werden die Bleche mit hohem Durchsatz verarbeitet, was kurze Verweilzeiten erforderlich macht, also nach Auftragen der Beschichtung eine Trocknung bei erhöhter Temperatur erforderlich macht, um die Beschichtungsmasse schnell belastbar zu machen.

Die Beschichtung der Substrate mit den Beschichtungsmassen erfolgt nach üblichen, dem Fachmann bekannten Verfahren, wobei man eine Beschichtungsmasse oder eine solche enthaltend Lackformulierung auf das zu beschichtende Substrat in der gewünschten Stärke aufbringt und gegebenenfalls trocknet. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen.

Weiterhin wird ein Verfahren zum Beschichten von Substraten offenbart, bei dem man das eine Beschichtungsmasse oder eine solche enthaltende Lackformulierung, gegebenenfalls mit weiteren lacktypischen Additiven und thermisch, chemisch oder strahlungshärtbaren Harzen versetzt, auf das Substrat aufbringt und gegebenenfalls trocknet, mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur und anschließend bei Temperaturen bis zu 160°C, bevorzugt zwischen 60 und 160 °C, besonders bevorzugt zwischen 100 und 160 °C, thermisch behandelt.
Die Strahlungshärtung erfolgt mit energiereichem Licht, z.B. UV-Licht oder Elektronenstrahlen. Die Strahlungshärtung kann bei höheren Temperaturen erfolgen. Bevorzugt ist dabei eine Temperatur oberhalb der T_{g} des strahlungshärtbaren Bindemittels.

Strahlungshärtung heißt hier die radikalische Polymerisation von polymerisierbaren Verbindungen infolge einer elektromagnetischen und/oder korpuskularen Strahlung, bevorzugt UV-Licht im Wellenlängenbereich von λ=200 bis 700 nm und/oder Elektronenstrahlung im Bereich von 150 bis 300 keV und besonders bevorzugt mit einer Strahlungsdosis von mindestens 80, bevorzugt 80 bis 3000 mJ/cm².

Neben einer Strahlungshärtung können noch weitere Härtungsmechanismen involviert sein, beispielsweise thermische-, Feuchtigkeits-, chemische und/oder oxidative Härtung, bevorzugt thermische und Strahlungshärtung und besonders bevorzugt Strahlungshärtung allein.

Die Beschichtungsmittel können nach den unterschiedlichsten Spritzverfahren, wie z.B. Luftdruck-, Airless- oder Elektrostatik-Spritzverfahren unter Verwendung von Ein- oder Zweikomponenten-Spritzanlagen, aber auch durch Spritzen, Spachteln, Rakeln, Bürsten, Rollen, Walzen, Gießen, Laminieren, Hinterspritzen oder Coextrudieren ein- oder mehrfach appliziert werden.

Die Beschichtungsstärke liegt in der Regel in einem Bereich von etwa 3 bis 1000 g/m² und vorzugsweise 10 bis 200 g/m².

Die Trocknung und Aushärtung der Beschichtungen erfolgt im allgemeinen unter normalen Temperaturbedingungen, d.h. ohne Erhitzung der Beschichtung. Die erfindungsgemäßen Mischungen können jedoch auch zur Herstellung von Beschichtungen eingesetzt werden, die nach Applikation bei erhöhter Temperatur, z.B. bei 40 - 250°C, vorzugsweise 40 - 150°C und insbesondere bei 40 bis 100°C getrocknet und ausgehärtet werden. Dies ist begrenzt durch die Thermostabilität des Substrats.

Weiterhin wird ein Verfahren zum Beschichten von Substraten offenbart, bei dem man die Beschichtungsmasse oder solche enthaltende Lackformulierungen, gegebenenfalls mit thermisch härtbaren Harzen versetzt, auf das Substrat aufbringt, trocknet, und anschließend mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur.

Das Verfahren zum Beschichten von Substraten kann auch so durchgeführt werden, daß nach dem Aufbringen der Beschichtungsmasse oder Lackformulierungen zunächst mit Elektronenstrahlen oder UV Belichtung unter Sauerstoff oder bevorzugt unter Inertgas bestrahlt wird, um eine Vorhärtung zu erzielen, anschließend bei Temperaturen bis zu 160 °C, bevorzugt zwischen 60 und 160 °C, thermisch behandelt und anschließend mit Elektronenstrahlen oder UV Belichtung unter Sauerstoff oder bevorzugt unter Inertgas endhärtet.

Gegebenenfalls kann, wenn mehrere Schichten des Beschichtungsmittels übereinander aufgetragen werden, nach jedem Beschichtungsvorgang eine Trocknung und/oder Strahlungshärtung erfolgen.

Als Strahlungsquellen für die Strahlungshärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Elektronenblitzeinrichtungen, wodurch eine Strahlungshärtung ohne Photoinitiator möglich ist, oder Excimerstrahler. Die Strahlungshärtung erfolgt durch Einwirkung energiereicher Strahlung, also UV-Strahlung oder Tageslicht, vorzugsweise Licht im Wellenlängenbereich von λ=200 bis 700 nm strahlt, besonders bevorzugt von λ=200 bis 500 nm und ganz besonders bevorzugt λ=250 bis 400 nm, oder durch Bestrahlung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm².

Selbstverständlich sind auch mehrere Strahlungsquellen für die Härtung einsetzbar, z.B. zwei bis vier.

Diese können auch in jeweils unterschiedlichen Wellenlängebereichen strahlen.

Die Trocknung und/oder thermische Behandlung kann auch zusätzlich zur oder anstelle der thermischen Behandlung durch NIR-Strahlung erfolgen, wobei als NIR-Strahlung hier elektromagnetische Strahlung im Wellenlängenbereich von 760 nm bis 2,5 µm, bevorzugt von 900 bis 1500 nm bezeichnet ist.

Die Bestrahlung kann gegebenenfalls auch unter Ausschluß von Sauerstoff, z. B. unter Inertgas-Atmosphäre, durchgeführt werden. Als Inertgase eignen sich vorzugsweise Stickstoff, Edelgase, Kohlendioxid, oder Verbrennungsgase. Des weiteren kann die Bestrahlung erfolgen, indem die Beschichtungsmasse mit transparenten Medien abgedeckt wird. Transparente Medien sind z. B. Kunststofffolien, Glas oder Flüssigkeiten, z. B. Wasser. Besonders bevorzugt ist eine Bestrahlung in der Weise, wie sie in der DE-A1 199 57 900 beschrieben ist.

Ein bevorzugter Gegenstand der vorliegenden Erfindung besteht in der Verwendung von Gemischen, enthaltend (U) und (V), bevorzugt erhältlich durch das erfindungsgemäße Verfahren, besonders bevorzugt erhalten durch das erfindungsgemäße Verfahren in Dentalmassen.

Bevorzugt enthalten solche Dentalmassen neben den Komponenten (U) und (V) noch anorganische Füllpartikel mit einem mittleren Teilchendurchmesser bis zu 1 µm, besonders bevorzugt bis zu 100 nm, ganz besonders bevorzugt 10 bis 50 nm.

Bei derartigen anorganischen Füllpartikeln kann es sich beispielsweise handeln um Kieselgele, Blancfixe, Kieselgur, Talkum, Calciumcarbonate, Kaolin, Bariumsulfat, Magnesiumsilikat, Aluminiumsilikat, kristallines Siliziumdioxid, amorphe Kieselsäure, Diamant, Granat, Bimsstein, Tripel, Siliciumcarbid, Schmirgel, Aluminiumoxide, wie beispielsweise Korund (α-Aluminiumoxid), Kieselgur, Sand (Schleifsande), Gips, Borcarbid, Boride, Carbide, Nitride, Zirkondioxid oder CeroxidMikrokugeln. Bevorzugt sind durch Hydrolyse von Siliciumtetrachlorid erhältliche Silikate wie Aerosil® der Fa. Evonik, Kieselerde, Talkum, Aluminiumsilikate, Magnesiumsilikate, Calciumcarbonate, besonders bevorzugt sind Silikate.

Diese anorganischen Materialien sind in Bezug auf die Summe der Komponenten (U) und (V) in Mengen von 0,1 - 70, bevorzugt 30 - 70 und besonders bevorzugt 50 - 70 Gew% anwesend.

Ferner können die erfindungsgemäßen Dentalmassen bezogen auf die Summe der Komponenten (U) und (V) zusätzlich 0 bis 10 Gew% mindestens eines Photoinitiators enthalten.

Photoinitiatoren können beispielsweise dem Fachmann bekannte Photoinitiatoren sein, z.B. solche in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SITA Technology Ltd, London, genannten.

In Betracht kommen solche Photoinitiatoren, wie sie beschrieben sind in WO 2006/005491 A1, Seite 21, Zeile 18 bis Seite 22, Zeile 2 (entspricht US 2006/0009589 A1, Absatz [0150]), was hiermit durch Bezugnahme Bestandteil der vorliegenden Offenbarung sei.

Geeignet sind auch nicht- oder wenig vergilbende Photoinitiatoren vom Phenylglyoxalsäureestertyp, wie in DE-A 198 26 712, DE-A 199 13 353 oder WO 98/33761 beschrieben.

Bevorzugt unter diesen Photoinitiatoren sind 2,4,6-Trimethylbenzoyldiphenylphosphinoxid, Ethyl-2,4,6-trimethylbenzoylphenylphosphinat, Bis-(2,4,6-trimethylbenzoyl)-phenylphosphinoxid, Benzophenon, 1-Benzoylcyclohexan-1-ol, 2-Hydroxy-2,2-dimethyl-acetophenon und 2,2-Dimethoxy-2-phenylacetophenon.

Die in dieser Schrift angegebenen %- und ppm-Angaben beziehen sich auf Gew.% und Gew.ppm, soweit nicht anders angegeben.

Die folgenden Beispiele sollen die Eigenschaften der Erfindung erläutern, ohne sie aber einzuschränken.

### Beispiele

### Beispiel 1, Vergleich:

In einem Glaskolben, ausgestattet mit Rührer, Thermometer und Kühler mit Druckausgleich wurden 100 g Isophoron-diisocyanat (IPDI), 30 g Trimethylolpropan (TMP) und 130 g Triethylenglykol-dimethacrylat (TEGDMA) bei 23°C vorgelegt. Nach Zugabe von 0,03 g Dibutylzinn-dilaurat wurde die Reaktionsmischung unter Rühren auf 50°C erwärmt, bei dieser Temperatur gehalten, und die Abnahme des NCO-Gehaltes titrimetrisch verfolgt. Nach Erreichen des NCO-Gehaltes von 7,2 Gew% wurden 65 g BASONAT® HI 100, gelöst in 65 g TEGDMA zugegeben, die Mischung auf 60°C erwärmt und 3 h bei dieser Temperatur weitergerührt. Das Produkt wies nun einen NCO-Gehalt von 6,7 Gew. % auf.

Das Produktgemisch wurde abgekühlt und analysiert.
NCO-Gehalt: 6,7 Gew.%
Viskosität bei 23°C: 28000 mPas
GPC: Mn = 850 g/mol, Mw = 6100 g/mol

### Beispiel 2:

In einem Glaskolben, ausgestattet mit Rührer, Thermometer und Kühler mit Druckausgleich wurden 100 g Isophoron-diisocyanat (IPDI), 30 g Trimethylolpropan (TMP), 130 g Triethylenglykol-dimethacrylat (TEGDMA) und 0,03 g 2,6-Di-tert.-butyl-p-kresol (BHT) bei 23°C vorgelegt. Nach Zugabe von 0,03 g Dibutylzinn-dilaurat wurde die Reaktionsmischung unter Rühren auf 50°C erwärmt, bei dieser Temperatur gehalten, und die Abnahme des NCO-Gehaltes titrimetrisch verfolgt. Nach Erreichen des NCO-Gehaltes von 7,4 Gew% wurden 65 g BASONAT® HI 100, gelöst in 65 g TEGDMA zugegeben, die Mischung auf 60°C erwärmt und 3 h bei dieser Temperatur weitergerührt. Das Produkt wies nun einen NCO-Gehalt von 6,7 Gew. % auf. Abschließend wurden 81 g Hydroxyethylmethacrylat (HEMA) zugegeben und das Produktgemisch bei 70°C weitergerührt, bis der NCO-Gehalt der Mischung 0 Gew.% betrug.
Das Produkt wurde abgekühlt und analysiert.
Viskosität bei 23°C: 49700 mPas
GPC: Mn = 1100 g/mol, Mw = 15000 g/mol

### Beispiel 3, Vergleich:

In einem Glaskolben, ausgestattet mit Rührer, Thermometer und Kühler mit Druckausgleich wurden 500 g Isophoron-diisocyanat (IPDI), 150 g Trimethylolpropan (TMP) und 650 g trockenes Butylacetat bei 23°C vorgelegt. Nach Zugabe von 0,1 g Dibutylzinn-dilaurat wurde die Reaktionsmischung unter Rühren auf 50°C erwärmt, bei dieser Temperatur gehalten, und die Abnahme des NCO-Gehaltes titrimetrisch verfolgt. Nach Erreichen des NCO-Gehaltes von 7,2 Gew% wurden 325 g BASONAT® HI 100, gelöst in 325 g trockenem Butylacetat zugegeben, die Mischung auf 60°C erwärmt und 3 h bei dieser Temperatur weitergerührt. Das Produkt wies nun einen NCO-Gehalt von 6,7 Gew. % auf. Abschließend wurden 81 g Hydroxyethylmethacrylat (HEMA) zugegeben und das Produktgemisch bei 70°C weitergerührt, bis der NCO-Gehalt der Mischung 0 Gew.% betrug.
Nun wurden 250g des Produktes in den 1000 ml Kolben eines Rotationsverdampfers überführt, um Butylacetat aus dem Reaktionsgemisch zu entfernen. Die Destillation des Lösemittels wurde bei einer Ölbadtemperatur von 70°C und vermindertem Druck durchgeführt, wobei über einen Zeitraum von 90 min der Druck im Rotationsverdampfer langsam auf 1 mbar gesenkt wurde. Anschließend wurde das Produkt noch 1 h bei 70°C und 1 mbar evakuiert. Nach dem Abkühlen war das Produkt fest. Trotz der Lösemittelabreicherung bei vermindertem Druck im Rotationsverdampfer war über dem Feststoff noch ein starker Butylacetat-Geruch wahrnehmbar. Der Versuch, den Feststoff per GPC zu analysieren, scheiterte, da das Urethanacrylat aufgrund von Teilvernetzung nicht mehr vollständig in DMAc zu lösen war.

### Beispiel 4, Vergleich:

In einem Glaskolben, ausgestattet mit Rührer, Thermometer und Kühler mit Druckausgleich wurden 100 g Isophoron-diisocyanat (IPDI) und 20,7 g Glycerin ohne Zugabe von Lösemittel oder Acrylat-gruppen enthaltendem Reaktivverdünner bei 23°C vorgelegt. Nach Zugabe von 0,25 g Dibutylzinn-dilaurat wurde die Reaktionsmischung unter Rühren auf 40°C erwärmt und die Abnahme des NCO-Gehaltes titrimetisch verfolgt.
Die Reaktion verlief exotherm, so dass die Temperatur auf 65°C anstieg und die Gegenkühlung aktiviert werden musste. Bei einem NCO-Gehalt von 12,5 Gew.% wurden 66g BASONAT® HI 100 zugegeben. Nach kurzer Reaktionszeit wurde das Reaktionsgemisch so viskos, dass der Rührer stehen blieb, die Gegenkühlung der Mischung versagte, die Innentemperatur auf 120°C anstieg und das Produkt vernetzte.

### Beispiel 5:

In einem Glaskolben, ausgestattet mit Rührer, Thermometer und Kühler mit Druckausgleich wurden 100 g IPDI, 30 g TMP und 130 g Hexandioldiacrylat (HDDA), nachstabilisiert mit 0,03 g Hydrochinon-monomethylether (MEHQ), bei 23°C vorgelegt. Nach Zugabe von 0,03 g Dibutylzinn-dilaurat wurde die Reaktionsmischung auf 50°C unter Rühren erwärmt, bei dieser Temperatur gehalten, und die Abnahme des NCO-Gehaltes titrimetrisch verfolgt. Nach Erreichen des NCO-Gehaltes von 7,3 Gew.% wurden 65 g BASONAT® HI 100, gelöst in 65 g mit MEHQ nachstabilisiertem HDDA zugegeben, die Mischung auf 60°C erwärmt und 3 h bei dieser Temperatur weitergerührt. Das Produkt wies nun einen NCO-Gehalt von 6,0 Gew. % auf. Abschließend wurden 42 g Hydroxyethylacrylat (HEA) zugegeben und das Produktgemisch bei 70°C weitergerührt, bis der NCO-Gehalt der Mischung 2 Gew.% betrug.
Das Produkt wurde beim Abkühlen fest.
NCO-Gehalt: 1,8 Gew.%
Glasübergangstemperatur Tg (°C): 13,5
GPC: Mn = 4500 g/mol, Mw = 49700 g/mol

### Beispiel 6:

In einem Glaskolben, ausgestattet mit Rührer, Thermometer und Kühler mit Druckausgleich wurden 80 g frisch destilliertes IPDI, 16,6 g Glycerin, 102 g Urethandimethacrylat (dem Reaktionsprodukt aus 2,2,4-Trimethylhexamethylendiisocyanat (TMDI) und HEMA im molaren Verhältnis von 1:2, UDMA), 102 g TEGDMA und 0,03 g 2,6-Di-tert.-butyl-p-kresol (BHT) bei 23°C vorgelegt. Dann wurde die Reaktionsmischung unter Rühren auf 45°C erwärmt, bei dieser Temperatur gehalten, und die Abnahme des NCO-Gehaltes titrimetrisch verfolgt. Bei einem NCO-Gehalt von 3,9 Gew.% wurden 52,8 g BASONAT® HI 100 zugegeben, die Mischung auf 60°C erwärmt und bei dieser Temperatur weitergerührt. Bei einem NCO-Gehalt von 4,9 Gew. % wurden nun 56 g HEMA zugegeben und das Produktgemisch bei 70°C weitergerührt, bis der NCO-Gehalt der Mischung 0 Gew.% betrug.
Das viskose Produkt wurde abgekühlt und analysiert.
Viskosität bei 60°C: 4180 mPas
GPC: Mn = 1670 g/mol, Mw = 19000 g/mol

### Beispiel 7:

In einem Glaskolben, ausgestattet mit Rührer, Thermometer und Kühler mit Druckausgleich wurden 100 g 2,4-Toluylen-diisocyanat (2,4-TDI), 38,5 g TMP, 138,5 g TEGDMA und 0,1 g 2,6-Di-tert.-butyl-p-kresol (BHT) bei 23°C vorgelegt. Dann wurde die Reaktionsmischung unter Rühren auf 45°C erwärmt, bei dieser Temperatur gehalten, und die Abnahme des NCO-Gehaltes titrimetrisch verfolgt. Bei einem NCO-Gehalt von 8,7 Gew.% wurden 78 g LUPRANAT® M 20 W, gelöst in 78 g TEGDMA zugegeben, die Mischung auf 60°C erwärmt und bei dieser Temperatur weitergerührt. Bei einem NCO-Gehalt von 8,8 Gew. % wurden nun 105 g HEA zugegeben und das Produktgemisch bei 70°C weitergerührt, bis der NCO-Gehalt der Mischung 0 Gew.% betrug.
Das viskose Produkt wurde abgekühlt und analysiert.
Viskosität bei 23°C: 39100 mPas
GPC: Mn = 1340 g/mol, Mw = 7400 g/mol

Die GPC-Analytik wurde mit Dimethylacetamid (DMAc) als mobiler Phase durchgeführt. Als Standard für die Molekulargewichtsbestimmung wurde Polymethylmethacrylat (PMMA) verwendet.

BASONAT™ HI 100: aliphatisches Polyisocyanurat der BASF SE, Isocyanatgehalt = 21,8 Gew. %, Viskosität bei 23°C = 3200 mPas.

LUPRANAT™ M 20 W: Polymer-MDI der BASF SE, Isocyanat-Gehalt = 31,5 Gew.%, Viskosität bei 23°C = 220 mPas.

## Patentansprüche

1. Verfahren zur Herstellung von strahlungshärtbaren, hochfunktionellen, hoch- oder hyperverzweigten Polyurethan(meth)acrylaten (U), umfassend die Reaktionsschritte
(i) Herstellen eines Additionsproduktes (A), das eine oder mehrere Isocyanatgruppen enthält und mindestens eine mit Isocyanat reaktive Gruppe enthält, durch Umsetzen eines
(I) (a1) Di- und/oder
(I) (a2) Polyisocyanats
mit
(b1) mindestens einer Verbindung mit mindestens drei mit Isocyanat reaktiven Gruppen
und/oder
(b2) mindestens einer Verbindung mit zwei mit Isocyanat reaktiven Gruppen,
wobei mindestens eine der Komponenten (a) oder (b) funktionelle Gruppen mit gegenüber den funktionellen Gruppen der anderen Komponente unterschiedlicher Reaktivität aufweist,
wobei die reaktiveren Gruppen aus den Verbindungen (a) und (b) in Schritt (i) im wesentlichen abreagieren, und
wobei das Umsetzungsverhältnis so gewählt wird, dass im Mittel das Additionsprodukt (A) mindestens eine mit Isocyanat reaktive Gruppe und eine oder mehrere Isocyanatgruppen enthält,
(ii) gegebenenfalls intermolekulare Additionsreaktion des Additionsprodukts (A) aus (i) zu einem Polyadditionsprodukt (P), das eine oder mehrere Isocyanatgruppen enthält und mindestens eine mit Isocyanat reaktive Gruppe enthalten kann,
(iii) gegebenenfalls Umsetzen des Additionsproduktes (A) aus (i) oder (P) aus (ii) mit mindestens einem Monoisocyanat und/oder mindestens einem Di- oder Polyisocyanat (I)(a1) oder (I)(a2) und/oder mindestens einem Di- oder Polyisocyanat (II), das vom Di- oder Polyisocyanat (I) verschieden ist, und
(iv) Umsetzen des Additionsproduktes (A) aus (i) und/oder des Polyadditionsproduktes (P) aus (ii) und/oder des Reaktionsproduktes aus (iii) mit mindestens einer Verbindung (c), die mindestens eine, bevorzugt genau eine gegenüber Isocyanat reaktive Gruppen und mindestens eine (Meth)acrylatgruppe aufweist,
in dem bei der Herstellung während des Reaktionsschrittes (i) mindestens ein Polyether(meth)acrylat oder ein (Meth)acrylat eines Di-, Tri- oder Tetraols (V) anwesend ist, das mindestens eine strahlungshärtbare Gruppe aufweist und das gegenüber den Reaktionspartnern aus Reaktionschritt (i) inert ist, wobei das mindestens eine Polyether(meth)acrylat oder (Meth)acrylat eines Di-, Tri- oder Tetraols (V) ein mittleres Molekulargewicht bis zu 1000 g/mol aufweist.

2. Verfahren gemäß Anspruch 1, **dadurch gekennzeichnet, daß** Di- oder Polyisocyanat (I) ausgewählt ist aus der Gruppe bestehend aus 2,4-Toluylendiisocyanat (2,4-TDI), 2,4'-Diphenylmethandiisocyanat (2,4'-MDI), Trüsocyanatotoluol, Isophorondiisocyanat (IPDI), 2-Butyl-2-ethylpentamethylendiisocyanat, 2-Isocyanatopropylcyclohexylisocyanat, 3(4)-Isocyanatomethyl-1-methylcyclohexyl-isocyanat, 1,4-Diisocyanato-4-methylpentan, 2,4'-Methylenbis-(cyclohexyl)diisocyanat und 4-Methylcyclohexan-1,3-diisocyanat (H-TDI).

3. Verfahren gemäß einem der vorstehenden Ansprüche, **dadurch gekennzeichnet, daß** das Di- oder Polyisocyanat (II) ausgewählt ist aus der Gruppe bestehend aus 2,4'- und 4,4'-Diphenylmethandiisocyanat, Mischungen aus Diphenylmethandiisocyanaten und höherkernigen Homologen des Diphenylmethandiisocyanats (Polymer-MDI), 1,3- und 1,4-Phenylendiisocyanat, 4-Isocyanatomethyl-1,8-octamethylendüsocyanat, Hexamethylendiisocyanat, Isocyanurat-, Uretdion-, Urethan-, Allophanat-, Iminooxadiazindion- oder Biuretgruppen aufweisenden Oligomeren aus Hexamethylendiisocyanat oder Isophorondiisocyanat (IPDI), Urethan-, Allophanat-, Carbodiimid- oder Uretonimingruppen aufweisenden Oligomeren aus MDI und Urethan-, Allophanat-, Carbodiimid- oder Uretonimingruppen aufweisenden Oligomeren aus TDI.

4. Verfahren gemäß einem der vorstehenden Ansprüche, **dadurch gekennzeichnet, daß** Komponente (b1) ausgewählt ist aus der Gruppe bestehend aus Glycerin, Trimethylolethan, Trimethylolpropan, 1,2,4-Butantriol, 1,2,6-Hexantriol, Pentaerythrit, Polyetherole auf Basis von Glycerin, Trimethylolpropan oder Pentaerythrit, Diethanolamin, Dipropanolamin und Tris(hydroxymethyl)amino-methan.

5. Verfahren gemäß einem der vorstehenden Ansprüche, **dadurch gekennzeichnet, daß** Komponente (b2) ausgewählt ist aus der Gruppe bestehend aus Ethylenglykol,1,2- und 1,3-Propandiol, 1,2-, 1,3-und 1,4-Butandiol, Ethanolamin, 1,2-Propanolamin, Mercaptoethanol, 4-Hydroxypiperidin und 1-Hydroxyethylpiperazin oder Polyetherolen

6. Verfahren gemäß einem der vorstehenden Ansprüche, **dadurch gekennzeichnet, daß** Komponente (c) ausgewählt aus der Gruppe bestehend aus 2-Hydroxyethylacrylat, 2-Hydroxyethylmethacrylat, 2- oder 3-Hydroxypropylacrylat, 2- oder 3-Hydroxypropylmethacrylat, 1,4-Butandiolmonoacrylat, 1,4-Butandiolmonomethacrylat, 1,2- oder 1,3-Diacrylat von Glycerin, Trimethylolpropandiacrylat, Pentaerythrittriacrylat, Ditrimethylolpropantriacrylat und Dipentaerythritpentaacrylat.

7. Verfahren gemäß einem der vorstehenden Ansprüche, **dadurch gekennzeichnet, daß** Verbindung (V) ausgewählt aus der Gruppe bestehend aus Ethylenglykoldi(meth)acrylat, Diethylenglykoldi(meth)acrylat, Triethylenglykoldi(meth)acrylat, Butandioldi(meth)acrylat, Hexandioldi(meth)acrylat, Trimethylolpropanmono-, di- oder tri(meth)acrylat, Pentaerythritmono-, di-, tri- oder tetra(meth)acrylat und 2,2-Bis-4-(2-hydroxy-3-methacryloxy) phenylpropan.

8. Verwendung von Gemischen, enthaltend strahlungshärtbare hochfunktionelle, hoch- oder hyperverzweigte Polyurethan(meth)acrylate (U) und Verbindungen (V) erhältlich nach einem der vorstehenden Ansprüche als Bausteine zur Herstellung von Lacken, Überzügen, Beschichtungsmassen, Formmassen und insbesondere in Dentalmassen.

9. Dentalmasse, enthaltend ein Gemisch strahlungshärtbare hochfunktionelle, hoch- oder hyperverzweigte Polyurethan(meth)acrylate (U) und Verbindungen (V) erhältlich nach einem der vorstehenden Ansprüche und gegebenenfalls zusätzlich anorganisches Füllmaterial.

## Claims

1. A process for preparing a radiation-curable, high-functionality, highly branched or hyperbranched polyurethane (meth)acrylate (U), comprising the reaction steps of
(i) preparation of an adduct (A), that comprises one or more isocyanate groups and comprises at least one isocyanate-reactive group by reaction of
(I) (a1) a diisocyanate and/or
(I) (a2) a polyisocyanate
with
(b1) at least one compound having at least three isocyanate-reactive groups
and/or
(b2) at least one compound having two isocyanate-reactive groups,
at least one of the components, (a) or (b), containing functional groups whose reactivity toward the functional groups of the other component is different,
the more reactive groups from the compounds (a) and (b) being substantially consumed by reaction in step (i), and
the reaction ratio being selected such that on average the adduct (A) comprises at least one isocyanate-reactive group and one or more isocyanate groups,
(ii)optionally, intermolecular addition reaction of the adduct (A) from (i) to give a polyadduct (P), that comprises one or more isocyanate groups and may comprise at least one isocyanate-reactive group,
(iii)optionally, reaction of the adduct (A) from (i) or (P) from (ii) with at least one monoiso-cyanate and/or with at least one diisocyanate or polyisocyanate (I) (a1) or (I) (a2) and/or with at least one diisocyanate or polyisocyanate (II) which is different from the diisocyanate or polyisocyanate (I), and
iv)reaction of the adduct (A) from (i) and/or of the polyadduct (P) from (ii) and/or of the reaction product from (iii) with at least one compound (c) which contains at least one, preferably precisely one, isocyanate-reactive group and at least one meth (acrylate) group,
in which, in the course of the preparation, during reaction step (i) at least one polyether (meth) acrylate or a (meth) acrylate of a di-, tri- or tetraol (V) is present which contains at least one radiation-curable group and which is inert toward the reactants from reaction step (i), the at least one polyether (meth) acrylate or (meth) acrylate of a di-, tri- or tetraol (V) having an average molecular weight of up to 1000 g/mol.

2. The process according to claim 1, wherein diisocyanate or polyisocyanate (I) is selected from the group consisting of 2,4-tolylene diisocyanate (2,4-TDI), 2,4'-diphenylmethane diisocyanate (2,4'-MDI), triisocyanatotoluene, isophorone diisocyanate (IPDI), 2-butyl-2-ethylpentamethylene diisocyanate, 2-isocyanatopropylcyclohexyl isocya-nate,3(4)-isocyanatemethyl-1-methylcyclohexyl iso-cyanate, 1,4-diisocyanato-4-methylpentane, 2,4'-methylenebis-(cyclohexyl) diisocyanate, and 4-methylcyclohexane 1,3-diisocyanate (H-TDI).

3. The process according to either of the preceding claims, wherein the diisocyanate or polyisocyanate (II) is selected from the group consisting of 2,4'- and 4,4'-diphenylmethane diisocyanate, mix-tures of diphenylmethane diisocyanates and more highly polycyclic homologs of diphenylmethane diisocyanate (polymeric MDI), 1,3- and 1,4-phenylene diisocyanate, 4-isocyanatomethyl-1,8-octamethylene diisocyanate, hexamethylene diiso-cyanate, oligomers of hexamethylene diisocyanate or isophorone diisocyanate (IPDI) that contain isocyanurate, uretdione, urethane, allophanate, iminooxadiazinedione or biuret groups, oligomers of MDI that contain urethane, allophanate, carbodiimide or uretonimine groups, and oligomers of TDI that contain urethane, allophanate, carbodiimide or uretonimine groups.

4. The process according to any of the preceding claims, wherein component (b1) is selected from the group consisting of glycerol, tri-methylolethane, trimethylolpropane, 1,2,4-butane-triol, 1,2,6-hexanetriol, pentaerythritol, poly-etherols based on glycerol, trimethylolpropane or pentaerythritol, diethanolamine, dipropanolamine, and tris(hydroxymethyl)aminomethane.

5. The process according to any of the preceding claims, wherein component (b2) is selected from the group consisting of ethylene glycol, 1,2- and 1,3-propanediol, 1,2-, 1,3- and 1,4-butanediol, ethanolamine, 1,2-propanolamine, mercaptoethanol, 4-hydroxypiperidine and 1-hydroxyethylpiperazine, or polyetherols.

6. The process according to any of the preceding claims, wherein component (c) is selected from the group consisting of 2-hydroxyethyl acrylate, 2-hydroxyethyl methacrylate, 2- or 3-hydroxypropyl acrylate, 2- or 3-hydroxypropyl methacrylate, 1,4-butanediol monoacrylate, 1,4-butanediol monomethacrylate, 1,2- or 1,3-diacrylate of glycerol, trimethylolpropane diacrylate, pentaerythritol triacrylate, ditrimethylolpropane triacrylate and dipentaerythritol pentaacrylate.

7. The process according to any of the preceding claims, wherein compound (V) is selected from the group consisting of ethylene glycol di(meth)acrylate, diethylene glycol di(meth)acrylate, triethylene glycol di(meth)acrylate, butanediol di(meth)acrylate, hexanediol di(meth)acrylate, trimethylolpropane mono-, di- or tri (meth) acrylate, pentaerythritol mono-, di-, tri- or tetra (meth) acrylate and 2,2-bis-4-(2-hydroxy-3-methacryloyloxy)phenylpropane.

8. The use of a mixture comprising radiation-curable, high-functionality, highly branched or hyperbranched polyurethane (meth) acrylate (U) and compound (V) as obtainable according to any of the preceding claims as a building block for producing a paint, covering, coating material or molding compound, and in particular in a dental composition.

9. A dental composition comprising a mixture of radiation-curable high-functionality, highly branched or hyperbranched polyurethane (meth) acrylate (U) and compound (V) as obtainable according to any of the preceding claims and optionally, in addition, inorganic filler material.

## Revendications

1. Procédé de fabrication de (méth)acrylates de polyuréthane hautement ou hyper-ramifiés, hautement fonctionnels, durcissables par rayonnement (U), comprenant les étapes de réaction suivantes :
(i) la fabrication d'un produit d'addition (A), qui contient un ou plusieurs groupes isocyanate et contient au moins un groupe réactif avec les isocyanates, par mise en réaction d'un
(I) (a1) di- et/ou
(I) (a2) polyisocyanate,
avec
(b1) au moins un composé contenant au moins trois groupes réactifs avec les isocyanates,
et/ou
(b2) au moins un composé contenant deux groupes réactifs avec les isocyanates,
au moins un des composants (a) ou (b) comprenant des groupes fonctionnels présentant une réactivité différente par rapport aux groupes fonctionnels des autres composants,
les groupes plus réactifs des composés (a) et (b) réagissant essentiellement à l'étape (i), et
le rapport de réaction étant choisi de sorte que le produit d'addition (A) contienne en moyenne au moins un groupe réactif avec les isocyanates et un ou plusieurs groupes isocyanate,
(ii) éventuellement la réaction d'addition intermoléculaire du produit d'addition (A) de (i) en un produit de polyaddition (P), qui contient un ou plusieurs groupes isocyanate et peut contenir au moins un groupe réactif avec les isocyanates,
(iii) éventuellement la mise en réaction du produit d'addition (A) de (i) ou (P) de (ii) avec au moins un monoisocyanate et/ou au moins un di- ou polyisocyanate (I) (a1) ou (I)(a2) et/ou au moins un di- ou polyisocyanate (II), qui est différent du di- ou polyisocyanate (I), et
(iv) la mise en réaction du produit d'addition (A) de (i) et/ou du produit de polyaddition (P) de (ii) et/ou du produit de réaction de (iii) avec au moins un composé (c), qui comprend au moins un, de préférence exactement un, groupe réactif avec les isocyanates et au moins un groupe (méth) acrylate,
selon lequel, lors de la fabrication, au moins un (méth) acrylate de polyéther ou un (méth) acrylate d'un di-, tri- ou tétraol (V) est présent pendant l'étape de réaction (i), qui comprend au moins un groupe durcissable par rayonnement et qui est inerte par rapport aux partenaires de réaction de l'étape de réaction (i), ledit au moins un (méth)acrylate de polyéther ou (méth)acrylate d'un di-, tri- ou tétraol (V) présentant un poids moléculaire moyen de jusqu'à 1 000 g/mol.

2. Procédé selon la revendication 1, **caractérisé en ce que** le di- ou polyisocyanate (I) est choisi dans le groupe constitué par le diisocyanate de 2,4-toluylène (2,4-TDI), le diisocyanate de 2,4'-diphénylméthane (2,4'-MDI), le triisocyanatotoluoène, le diisocyanate d'isophorone (IPDI), le diisocyanate de 2-butyl-2-éthylpentaméthylène, l'isocyanate de 2-isocyanatopropylcyclohexyle, l'isocyanate de 3(4)-isocyanatométhyl-1-méthylcyclohexyle, le 1,4-diisocyanato-4-méthylpentane, le diisocyanate de 2,4'-méthylènebis-(cyclohexyle) et le 1,3-diisocyanate de 4-méthyl-cyclohexane (H-TDI).

3. Procédé selon l'une quelconque des revendications précédentes, **caractérisé en ce que** le di- ou polyisocyanate (II) est choisi dans le groupe constitué par le diisocyanate de 2,4'- et 4,4'-diphénylméthane, les mélanges de diisocyanates de diphénylméthane et les homologues à nombre de noyaux plus élevé de diisocyanate de diphénylméthane (MDI polymère), le diisocyanate de 1,3- et 1,4-phénylène, le diisocyanate de 4-isocyanatométhyl-1,8-octaméthylène, le diisocyanate d'hexaméthylène, les oligomères de diisocyanate d'hexaméthylène ou de diisocyanate d'isophorone (IPDI) comprenant des groupes isocyanurate, uretdione, uréthane, allophanate, iminooxadiazine-dione ou biuret, les oligomères de MDI comprenant des groupes uréthane, allophanate, carbodiimide ou urétonimine et les oligomères de TDI comprenant des groupes uréthane, allophanate, carbodiimide ou urétonimine.

4. Procédé selon l'une quelconque des revendications précédentes, **caractérisé en ce que** le composant (b1) est choisi dans le groupe constitué par la glycérine, le triméthyloléthane, le triméthylolpropane, le 1,2,4-butanetriol, le 1,2,6-hexanetriol, la pentaérythrite, les polyétherols à base de glycérine, de triméthylolpropane ou de pentaérythrite, la diéthanolamine, la dipropanolamine et le tris(hydroxyméthyl)aminométhane.

5. Procédé selon l'une quelconque des revendications précédentes, **caractérisé en ce que** le composant (b2) est choisi dans le groupe constitué par l'éthylène glycol, le 1,2- et 1,3-propanediol, le 1,2-, 1,3- et 1,4-butanediol, l'éthanolamine, la 1,2-propanolamine, le mercaptoéthanol, la 4-hydroxypipéridine et la 1-hydroxyéthylpipérazine ou les polyétherols.

6. Procédé selon l'une quelconque des revendications précédentes, **caractérisé en ce que** le composant (c) est choisi dans le groupe constitué par l'acrylate de 2-hydroxyéthyle, le méthacrylate de 2-hydroxyéthyle, l'acrylate de 2- ou 3-hydroxypropyle, le méthacrylate de 2- ou 3-hydroxypropyle, le monoacrylate de 1,4-butanediol, le monométhacrylate de 1,4-butanediol, le 1,2- ou 1,3-diacrylate de glycérine, le diacrylate de triméthylolpropane, le triacrylate de pentaérythrite, le triacrylate de ditriméthylolpropane et le pentaacrylate de dipentaérythrite.

7. Procédé selon l'une quelconque des revendications précédentes, **caractérisé en ce que** le composé (V) est choisi dans le groupe constitué par le di(méth)acrylate d'éthylène glycol, le di(méth)acrylate de diéthylène glycol, le di(méth)acrylate de triéthylène glycol, le di(méth)acrylate de butanediol, le di(méth)acrylate d'hexanediol, le mono-, di- ou tri(méth)acrylate de triméthylolpropane, le mono-, di-, tri- ou tétra(méth)acrylate de pentaérythrite et le 2,2-bis-4-(2-hydroxy-3-méthacryloxy)phénylpropane.

8. Utilisation de mélanges, contenant des (méth)acrylates de polyuréthane hautement ou hyper-ramifiés, hautement fonctionnels, durcissables par rayonnement (U) et des composés (V), pouvant être obtenus selon l'une quelconque des revendications précédentes en tant que constituants pour la fabrication de vernis, de revêtements, de matériaux de revêtement, de matériaux de moulage et notamment dans des matériaux dentaires.

9. Matériau dentaire, contenant un mélange de (méth)acrylates de polyuréthane hautement ou hyper-ramifiés, hautement fonctionnels, durcissables par rayonnement (U) et de composés (V), pouvant être obtenu selon l'une quelconque des revendications précédentes, et éventuellement en outre un matériau de remplissage inorganique.
